# EUROPEAN PATENT SPECIFICATION

(11) **EP 3 562 511 B1**
(45) Date of publication and mention of the grant of the patent: **06.08.2025**
(21) Application number: 17886028.4
(22) Date of filing: 29.12.2017
(51) Int. Cl.: A61K 9/127, A61K 9/51, C12N 15/11, C12N 15/113, A61P 9/10

(54) **RNAI THERAPIES FOR CEREBRAL ISCHEMIA**
RNAI-THERAPIEN FÜR ZEREBRALE ISCHÄMIE
THÉRAPIES À BASE D'ARNI POUR ISCHÉMIE CÉRÉBRALE

(30) Priority: 30.12.2016 US 201662440882 P
(43) Date of publication of application: 06.11.2019
(73) Proprietor: Vivibaba, Inc, Cheyenne, WY 82001 (US)
(72) Inventor: WEN, Jing, Culver City CA 90230 (US)
(74) Representative: Vossius & Partner Patentanwälte Rechtsanwälte mbB
(86) International application number: PCT/US2017/069064
(87) International publication number: WO 2018/126198

(56) References cited:
- WO-A2-2005/042719
- US-A1- 2015 071 999
- US-A1- 2015 071 999
- CHAOYONG LIU ET AL: "Efficient Delivery of Therapeutic miRNA Nanocapsules for Tumor Suppression", ADVANCED MATERIALS, vol. 27, no. 2, 14 November 2014 (2014-11-14), pages 292 - 297, XP055730437, ISSN: 0935-9648, DOI: 10.1002/adma.201403387
- YUBIN MENG ET AL: "An injectable miRNA-activated matrix for effective bone regeneration in vivo", JOURNAL OF MATERIALS CHEMISTRY B, vol. 4, no. 43, 3 October 2016 (2016-10-03), GB, pages 6942 - 6954, XP055730398, ISSN: 2050-750X, DOI: 10.1039/C6TB01790H
- BEN BULLER ET AL: "MicroRNA-21 protects neurons from ischemic death : miR-21 protects neurons from ischemic death", FEBS JOURNAL, vol. 277, no. 20, 14 September 2010 (2010-09-14), GB, pages 4299 - 4307, XP055730536, ISSN: 1742-464X, DOI: 10.1111/j.1742-4658.2010.07818.x
- AN SAI, ET AL.: "Brain-targeting delivery for RNAi neuroprotection against cerebral ischemia reperfusion injury", BIOMATERIALS, vol. 34, no. 35, 2013, pages 8949 - 8959, XP028699871

## Description

### BACKGROUND

Ischemia, resulting from occlusion of arterial blood supply, can lead to severe imbalance of metabolic supply and demand, causing hypoxia and dysfunction of the downstream tissues. Brain ischemia, particularly, is the most difficult to treat, causing high rate of death and disability (as the second leading cause of death and the third leading cause of disability in the world). Current treatments for brain ischemia are mainly focused on improving or restoring the flood flow, improving perfusion to the affected brain region, and inducing regeneration of affected brain tissues. In this context, thrombolytic drugs are commonly administrated through intravenous or intra-arterial infusion to dissolve blood clots in thrombolysis hours after actual ischemia stroke. For the regeneration purpose, neurotrophic drugs were tested in small trials. Their efficacy, however, remains inconclusive. Although positive results were demonstrated for intravenous transplantation of autologous bone marrow cells or intracerebral transplantation of neural stem cells, such transplantation strategies require costly and complicated procedures.

Buller et al. (FEBS J (2010) 277:4299-4307) disclose that overexpression of miR-21 protects against ischemic neuronal death, and that these findings suggest that miR-21 may be an attractive therapeutic molecule for treatment of stroke.

Systemic delivery of microRNA, a class of molecules that regulate the expression of cellular proteins associated with angiogenesis, cell growth, proliferation and differentiation, holds great promise for the treatment of brain ischemia. However, their therapeutic efficacy has been hampered by poor delivery efficiency of microRNA. MicroRNA (miRNA), a family of small non-coding RNAs with 21 to 25 nucleotides in length, regulates the expression of various cellular proteins that are associated with angiogenesis, cell growth, proliferation and differentiation. Administration of miRNA could enhance blood-vessel growth and help to recover the function of damaged tissues, which may provide an effective strategy for treatment of ischemic disease. Indeed, it has been demonstrated that local administration of miRNA to sequestered anatomical sites could facilitate function recovery in models of brain ischemia; however, such an invasive method requires multiple injections to the target sites or anatomically distinct regions. Systemic administration of miRNA, on the other hand, will be highly acceptable in the clinic practices, but remains ineffective for the treatment of brain ischemia. To date, viral and non-viral vectors (e.g., lipids, peptides, cyclodextrin and polymers) have been extensively explored for systematic delivery of miRNA. However, systemic delivery of miRNA for brain-tissue regeneration has not been demonstrated yet.

Therefore, there is great need for a more effective and efficient delivery method to treat ischemia and other diseases.

### SUMMARY

Provided herein are compositions and therapeutics for the treatment of diseases, including diseases characterized by gene dysregulation, based on nucleic acid nanocapsules. The disclosed compositions enable their effective delivery to the disease sites in the brain, such as sites of infarcts or other vascular injury. Exemplified by microRNA-21, intravenous injection of the nanocapsules into a rat model of cerebral ischemia could effectively ameliorate the infarct volume, neurological deficit and histopathological severity.

### BRIEF DESCRIPTION OF THE DRAWINGS

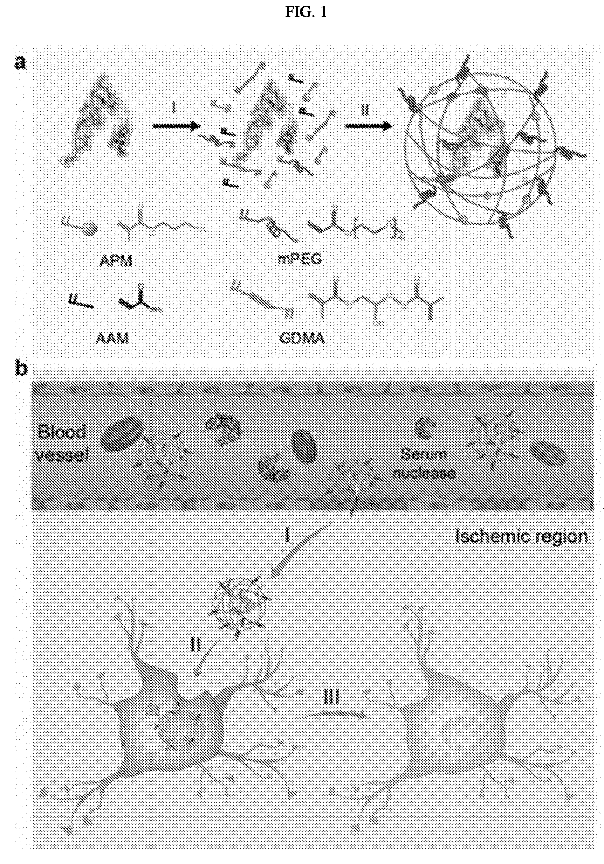

**FIG. 1** contains two panels, namely FIG. 1A and 1B, which depicts a schematic illustration of the synthesis of miRNA nanocapsules and their systemic delivery for brain ischemia therapy. Synthesis of miRNA nanocapsules include Step I for enrichment of the monomers and crosslinker molecules around the miRNA molecules and Step I for formation of n(miRNA) upon growth of a thin polymer shell around the miRNA molecules (FIG. 1A). Systemic delivery of n(miRNA) for ischemia therapy include (I) Accumulation of n(miRNA) in ischemic region of brain tissue due to increased permeability of the blood vessels; (II) Cellular uptake of n(miRNA) and release of miRNA in the cytosol; and (III) Regeneration of the neurons promoted by miRNA delivered (FIG.1B).

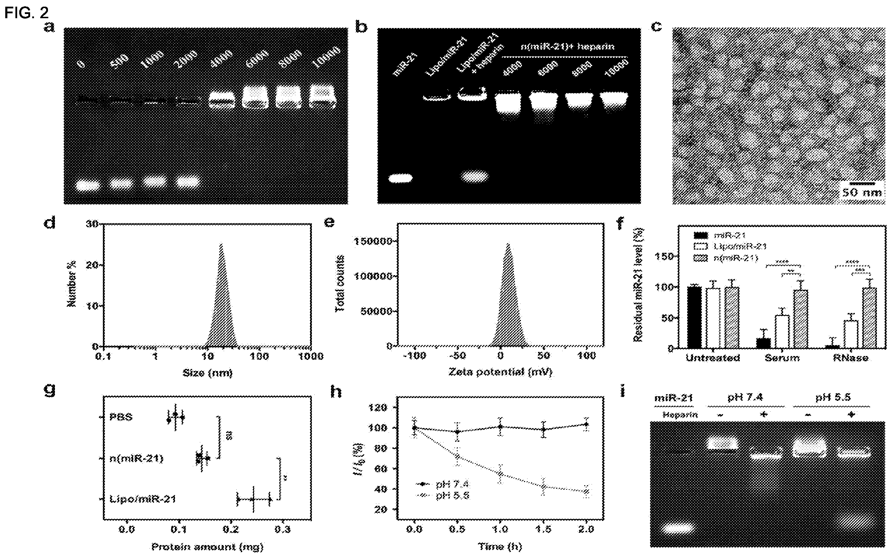

**FIG. 2** contains nine panels, namely FIG. 2A to 2I, which depicts structure, size, stability, protein adsorption, and degradation kinetics of miR-21 nanocapsules. Agarose gel electrophoresis of n(miR-21) synthesized at various monomer/miRNA (M/R) molar ratios (FIG. 2A). Stability of Lipo/miR-21 complex and n(miR-21) synthesized at various M/R molar ratios after incubation with 10 mg/mL heparin is shown in FIG. 2B. Transmission electron microscopic (TEM) image (FIG. 2C), hydrodynamic size (FIG. 2D) and zeta potential (FIG. 2E) of n(miR-21) synthesized with a M/R ratio of 6000:1 are shown here. The hydrodynamic size and zeta potential were measured by dynamic light scattering (DLS) with scattering angle at 173°. Residual miR-21 levels of native miR-21, Lipo/miR-21, and n(miR-21) after incubation with mouse whole serum or RNase at 37°C for 2h, respectively, are compared (FIG. 2F). Residual miRNAs were then extracted with chloroform/ 0.1% SDS-0.5 M NaCl and measured by qRT-PCR. Data are presented as mean ± SD (n=3). ***P*<0.01, ****P*<0.001 and *****P*<0.0001 (Turkey's post-test following ordinary one-way ANOVA). Quantitative measurements of serum proteins adsorbed by n(miR-21) and Lipo/miR-21 after incubation with mouse whole serum is shown in FIG. 2G. Data are presented as mean ± SD (n=3). ***P*<0.01 (Turkey's post-test following ordinary one-way ANOVA). Relative scattering light intensity (*I*/*I₀* × 100%) variation of n(miR-21) after incubation in 50 mM sodium acetate buffer (pH 5.5) and 50 mM HEPES buffer (pH 7.4) at 37°C for 2h are compared, where *I₀* is the initial n(miR-21) scattering light intensity, and *I* is the scattering light intensity measured at different time points with a detection angle of 173° (FIG. 2H). Data are normalized to facilitate direct comparison and presented as mean ± SD (*n*=5). Agarose gel electrophoresis of n(miR-21) after incubation in 50 mM sodium acetate buffer (pH 5.5) and 50 mM HEPES buffer (pH 7.4) at 37°C for 2h is shown in FIG. 2I. The notation "-" represents "without 1 mg/mL heparin incubation" and "+" represents "with 1 mg/mL heparin incubation", respectively.

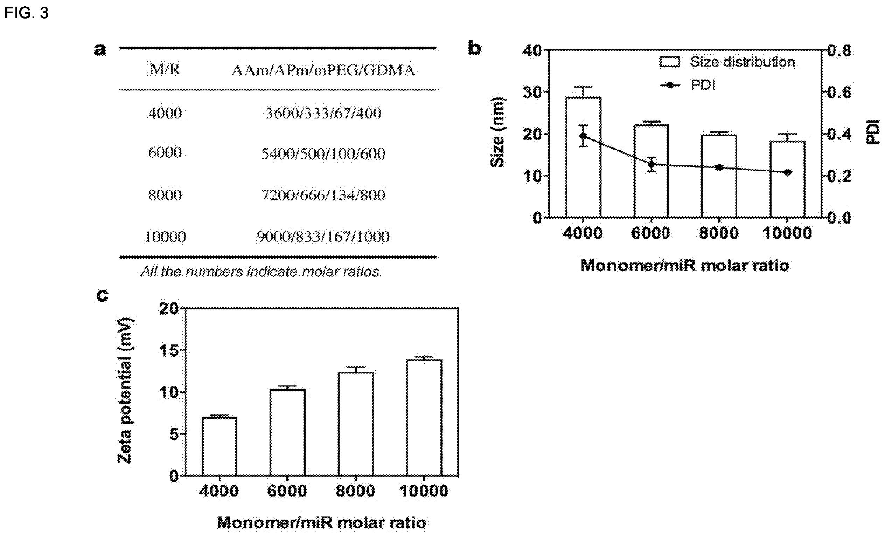

**FIG. 3** contains three panels, namely FIG. 3A to 3C, which shows the characteristics of nanocapsules synthesized with various monomers to RNA (M:R) molar ratios, including synthesis parameters (FIG. 3A), size distribution (FIG. 3B) and zeta potential trend (FIG. 3C) of n(miR-21) in a series of monomers to RNA molar ratios. Data in FIG. 3B and FIG. 3C are presented as mean ± SEM (n=3).

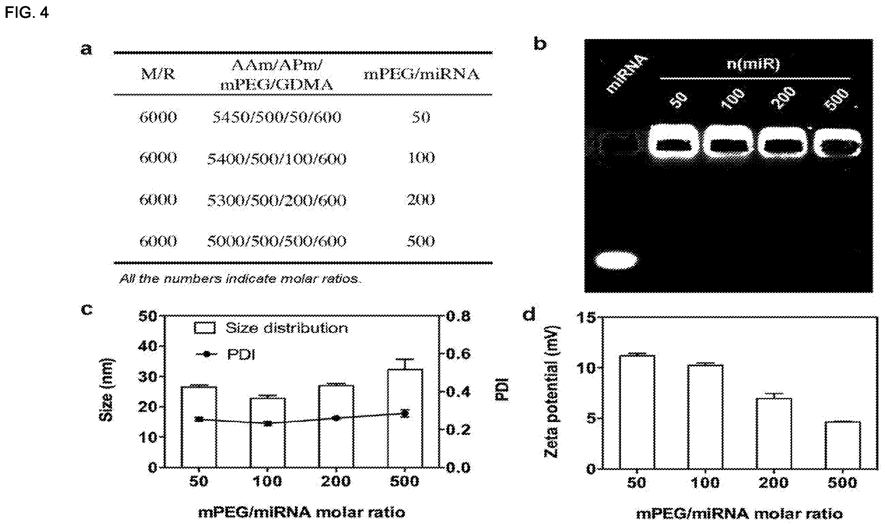

**FIG. 4** contains four panels, namely FIG. 4A to 4D, which shows the characteristics of nanocapsules synthesized at a M:R ratio of 6000:1 with various mPEG/miRNA molar ratios, including synthesis parameters (FIG. 4A), agarose gel electrophoresis (FIG. 4B), size distribution and PDI (FIG. 4C) and zeta potential trend (FIG. 4D) of n(miR-21). Data in FIG. 4C and FIG. 4D are presented as mean ± SEM (n=3).

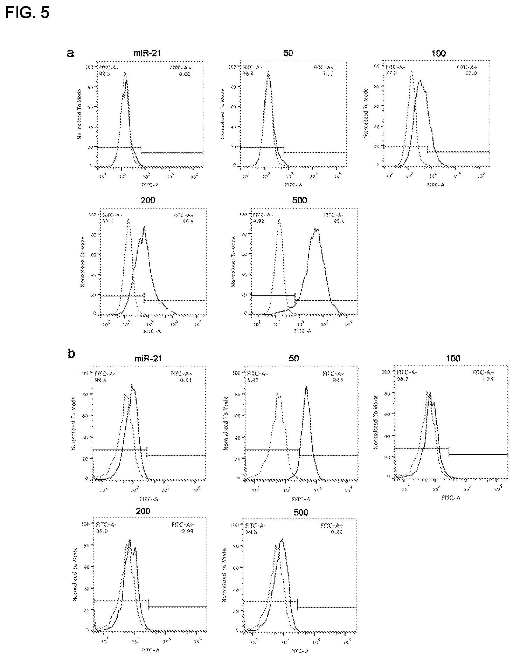

**FIG. 5** contains two panels, namely FIG. 5A and 5B, which depicts a fluorescence-assisted cell sorting analysis of C6 cells (FIG. 5A) and J774A.1 cells (FIG. 5B) treated with miR-21 and n(miR-21) synthesized at a M:R ratio of 6000:1 with various mPEG/miRNA molar ratios. C6 and J774A.1 cells were treated with samples at 50 nM miRNA for 4 hrs at 37 °C. MiR-21 was labeled with FITC. The trypan blue quenching assay was performed before measurement.

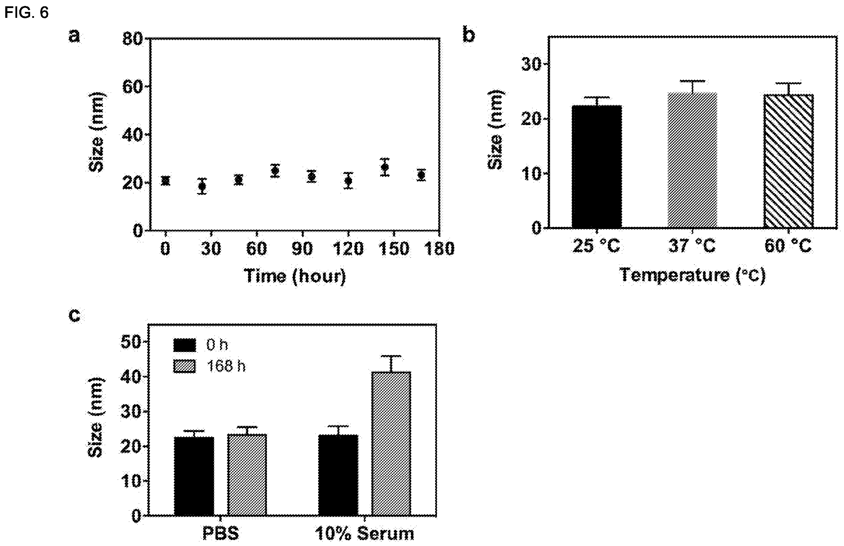

**FIG. 6** contains three panels, namely FIG. 6A to 6C, which depicts characteristics of synthesized n(miR-21), including the stability of n(miR-21) synthesized at a monomer/miRNA molar ratio of 6000 in PBS (pH 7.4) at 4 °C (FIG. 6A), the temperaturedependent size variation of n(miR-21) in the PBS buffer with different temperatures at 25, 37, and 60 °C (FIG. 6B), and the hydrodynamic size of n(miR-21) in either the presence or absence of 10% serum containing PBS buffer (FIG. 6C). All data in three panels are presented as mean ± SEM (n=3).

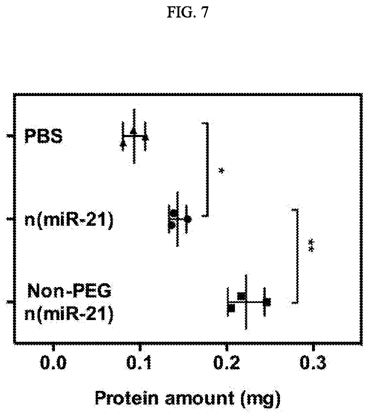

**FIG. 7** depicts a quantitative measurements of serum proteins adsorbed by n(miR-21) synthesized at a M:R ratio of 6000:1 and n(miR-21) synthesized at a M:R ratio of 6000:1 without mPEG (non-PEG n(miR-21)) after incubation with mouse whole serum. Data are presented as mean ±SD (n=3). **P*<0.05 and **P*<0.01 (Turkey's post-test following ordinary one-way ANOVA).

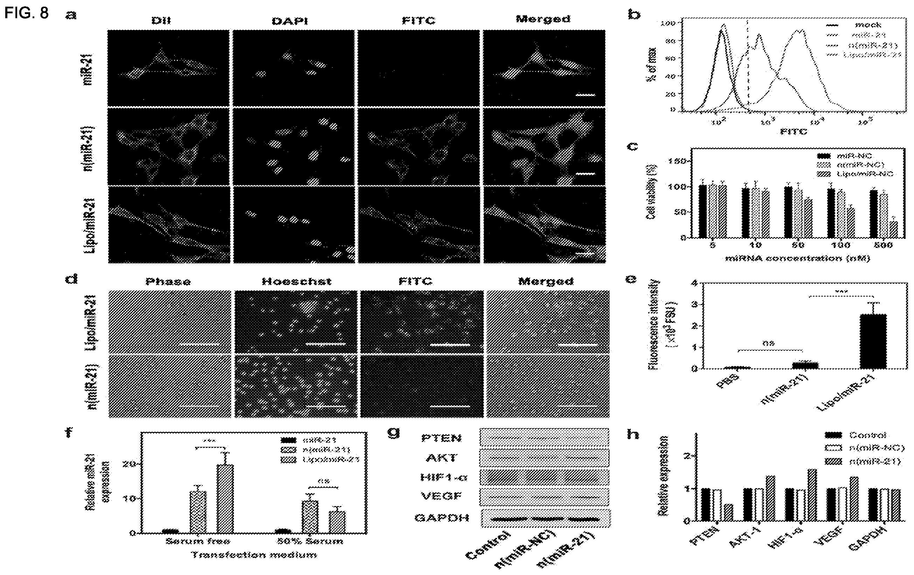

**FIG. 8** contains eight panels, namely FIG. 8A to 8H, which depicts cytotoxicity, cellular internalization efficiency, non-specific phagocytosis and intracellular activity of the miRNA nanocapsules. Confocal microscopic images of intracellular distribution of miR-21, n(miR-21) and Lipo/miR-21 in C6 cells after 4-hour incubation at 37°C is shown in FIG. 8A. Scale bar: 20 µm. The miR-21 was labeled with FITC. The cells were counterstained with DAPI (for nuclei), DiI (for cell membrane). Scale bar: 20 µm. Fluorescence-assisted cell sorting (FACS) analysis of C6 cells after 4-hour incubation with miR-21 and n(miR-21) at 37°C is shown in FIG. 8B. The miR-21 was labeled with FITC. The trypan blue quenching assay was performed before measurement. Cell viability assays of n(miR-NC) in C6 cells after 4-hour incubation at 37°C is shown in FIG. 8C. Data are presented as mean ± SD (n=5). Fluorescence images of J774A.1 mouse macrophages after 1-hour incubation at 37°C with n(miR-21) and Lipo/miR-21 is shown in FIG. 8D. MiR-21 was labeled with FITC. The cells were stained with Hoechst 33342 for imaging the nuclei. Scale bar: 100 µm. Histogram is compared for the mean fluorescent intensity accessed from FACS analysis of the macrophages after incubating with n(miR-21) and Lipo/miR-21 (FIG. 8E). Data are presented as mean ± SD (n=3). ****P*<0.001 (Turkey's post-test following ordinary one-way ANOVA). Quantitative real-time PCR assay was performed to compare miR-21 levels in C6 cells treated with various samples in serum-free medium or 50% human serum medium (FIG. 8F). Data are presented as mean ± SD (*n*=3). **P*<0.05 and ***P*<0.01 (Turkey's post-test following ordinary two-way ANOVA). Western blot analysis of protein expression in C6 cells 48 hours after sample administration is shown in FIG. 8G. Quantification of protein expression levels are shown as normalized gray values obtained from FIG. 8G by ImageJ software (FIG. 8H).

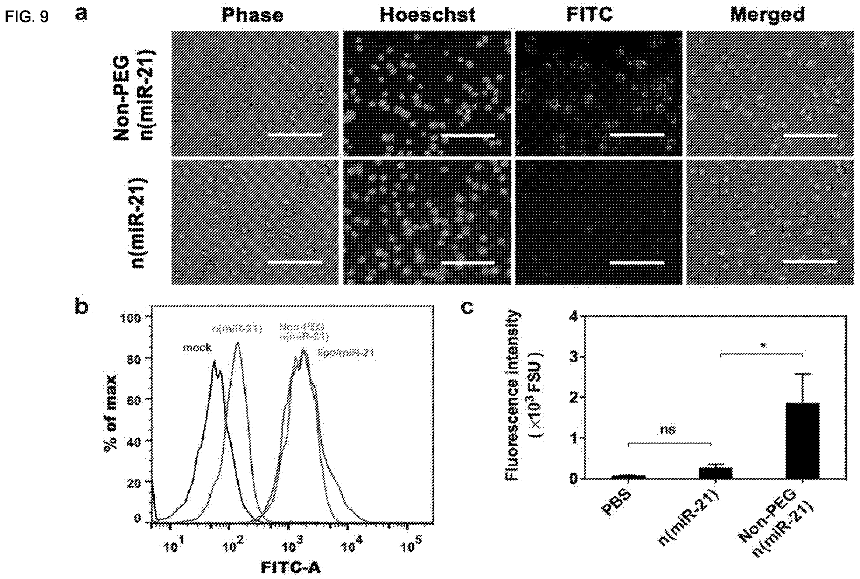

FIG. 9 contains three panels, namely FIG. 9A to 9C, which depicts characterization of J774A.1 mouse macrophages after n(miR-21) treatment. Fluorescence images of J774A.1 mouse macrophages 1-hour after incubation with n(miR-21) and non-PEG n(miR-21) were compared in FIG. 9A. Cells were stained with Hoechst 33342 for imaging the nuclei. MiR-21 was labeled with FITC. Scale bar: 100 µm. FACS analysis of the J774A.1 macrophages after incubating with n(miR-21), non-PEG n(miR-21) and Lipo/miR-21 was performed (FIG. 9B). Histogram was performed to compare the mean fluorescent intensity accessed from FACS analysis of the macrophages after incubating with n(miR-21) and non-PEG n(miR-21) (FIG. 9C). Data are presented as mean ± SD (*n*=3). **P*<0.05 (Turkey's post-test following ordinary one-way ANOVA).

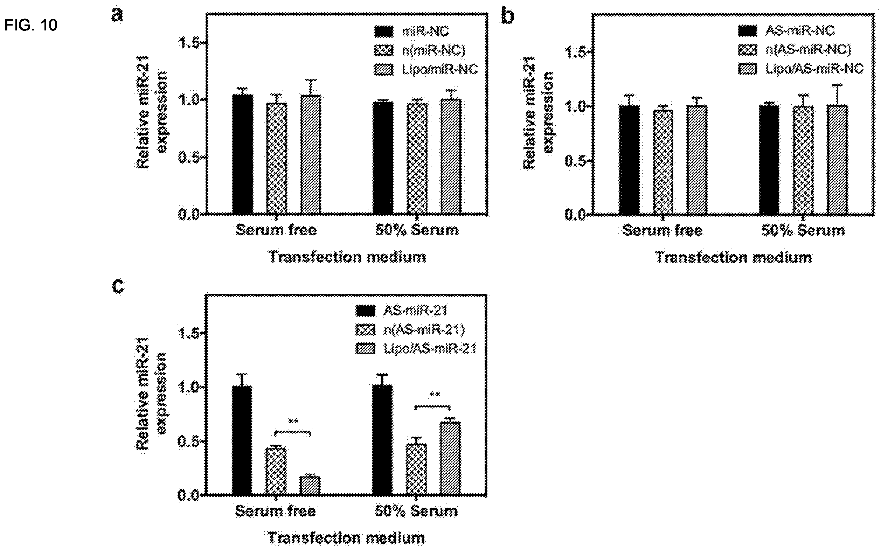

**FIG. 10** contains three panels, namely FIG. 10A to 10C, which depicts quantitative real-time PCR assay of miR-21 levels in C6 cells treated with various samples in serum-free medium or 50% human serum medium. Data are presented as mean ± SD (*n*=3). ***P*<0.01 (Turkey's post-test following ordinary two-way ANOVA).

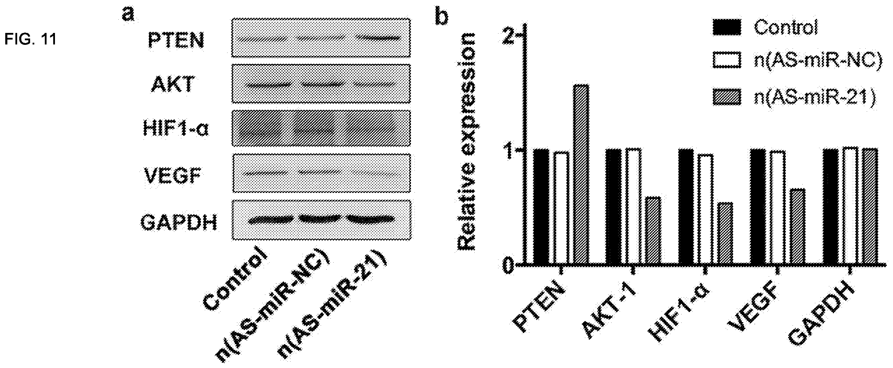

**FIG. 11** contains two panels, namely FIG. 11A and 11B, which depicts western blot analysis (FIG. 11A) of protein expression in C6 cells 48 hours after sample administration and the normalized protein expression levels obtained from quantitative analysis by ImageJ software (FIG. 11B).

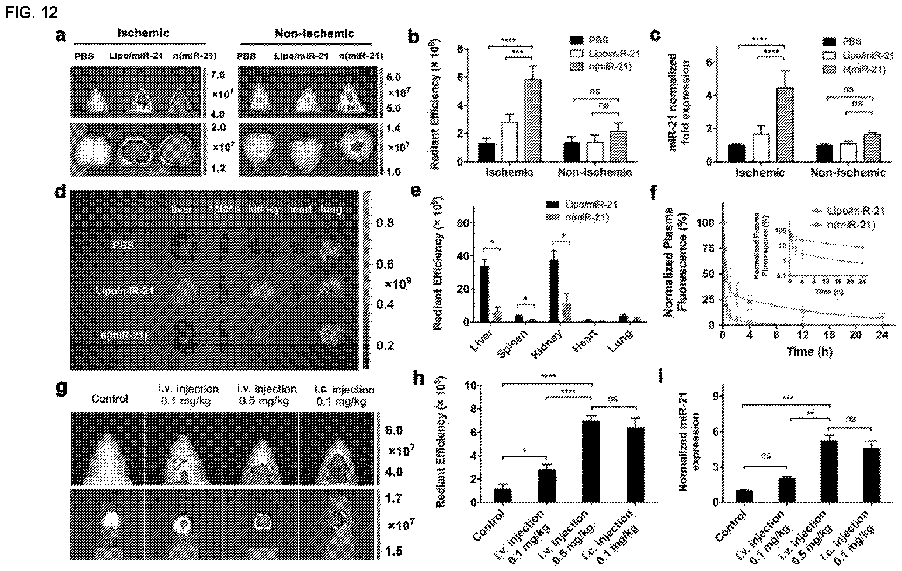

**FIG. 12** contains nine panels, namely FIG. 12A to 12I, which depicts biodistribution, pharmacokinetics, and the administration route comparison of the miRNA nanocapsules. *In vivo* fluorescence images are show in FIG. 12A for the ischemic (transient focal cerebral ischemia) model rats or non-ischemic rats (upper), and *ex vivo* fluorescence images of the collected brain tissues (lower) 24 hours after intravenous (*i.v.*) administration of the Cy5.5 labeled miR-21 nanocapsule (n(Cy5.5-miR-21)) at a dosage of 0.5 mg/kg miR-21, and equal volume PBS was administrated as control. ROI analysis of fluorescent signals (FIG. 12B) and mature miR-21 levels (FIG. 12C) of the collected brain tissues were performed. Data are presented as mean ± SD (*n*=5). **P*<0.05 and ***P*<0.001 (Turkey's post-test following ordinary two-way ANOVA). *Ex vivo* fluorescence images (FIG. 12D) and ROI analysis of fluorescent signals (FIG. 12E) show the collected major organs 24 hours after injection of Lipo/ Cy5.5-miR-21 and n(Cy5.5-miR-21) at a dosage of 0.5 mg miR-21 per kg rat, and equal volume PBS was administrated as control. Data are presented as mean ± SD (*n*=5). **P*<0.05 (two-tailed unpaired Student's t-test). Blood circulation profiles of n(Cy5.5-miR-21) in rats after intravenous injection of Lipo/ Cy5.5-miR-21 and n(Cy5.5-miR-21) at a dosage of 0.5 mg/kg miR-21 are compared in FIG. 12F. Inset: Enlarged blood circulation profiles for the initial 2 hours, which is plotted on a logarithmic y axis. Data are presented as mean ± SEM (*n*=5)*. In vivo* fluorescence images of the ischemic model rats (upper), and *ex vivo* images of the collected brains (lower) 24 hours after intracarotid (*i.c.*) or *i.v*. injection of different dosages of n(Cy5.5-miR-21) are compared in FIG. 12G. ROI analysis of fluorescent signals (FIG. 12H) and mature miR-21 levels (FIG. 12I) were measured for the collected brain tissues in FIG. 12G. Data are presented as mean ± SEM (*n*=5). The significance levels are **P*<0.01 and ***P*<0.001 (Turkey's post-test following ordinary one-way ANOVA).

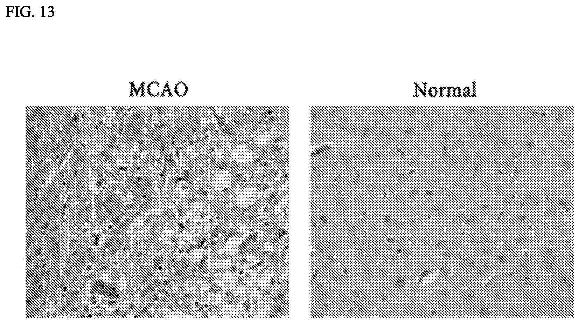

**FIG. 13** depict representative H&E stained images of brain tissues obtained from the sacrificed MCAO/R or Normal rats.

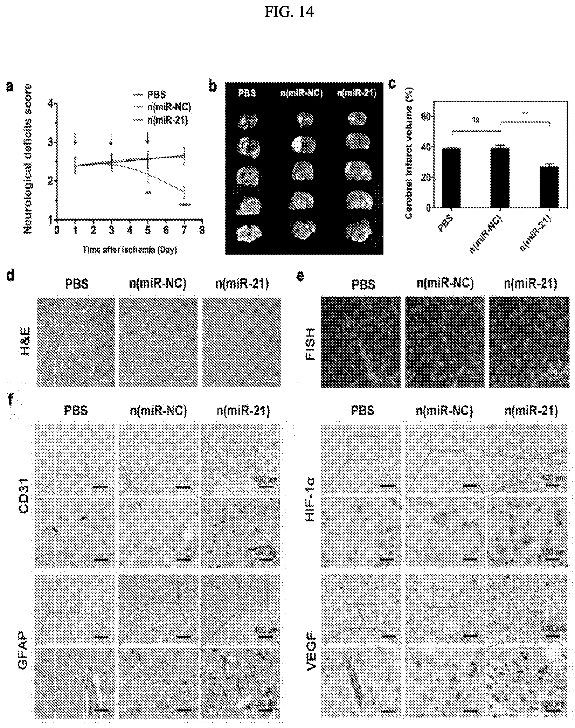

**FIG. 14** contains six panels, namely FIG. 14A to 14F, which depicts efficacy of the miRNA nanocapsules as therapeutic for transient focal cerebral ischemia. Neurologic function of transient focal cerebral ischemia model rats after samples administration is compared in FIG. 14A. The arrows indicate the sample administration time point. Neurologic deficit score was tested every other day before each injection. Data are presented as mean ± SEM. (*n*=10). **P*<0.05 and ***P*<0.01 (Bonferroni's multiple comparisons test following two-way RM ANOVA). TTC staining of brain tissues obtained from the sacrificed rats at day 7 is shown in FIG. 14B. Quantified cerebral infarction volume from FIG. 14B was measured (FIG. 14C). Data are presented as mean ± SEM (*n*=5). **P*<0.05 and ***P* <0.01. (Turkey's post-test following ordinary one-way ANOVA). Representative HE staining images of infarct area are shown in FIG. 14D. Scale bar: 400 µm. Representative fluorescence *in situ* hybridization detection of miR-21 in the infarct area is measured (FIG. 14E). Blue color represents DAPI and red represents miR-21. Scale bar: 100 µm. Representative immunohistochemistry images of infarct area are shown in FIG. 14F. Scale bar: 400 µm (upper) and Scale bar: 150 µm (lower).

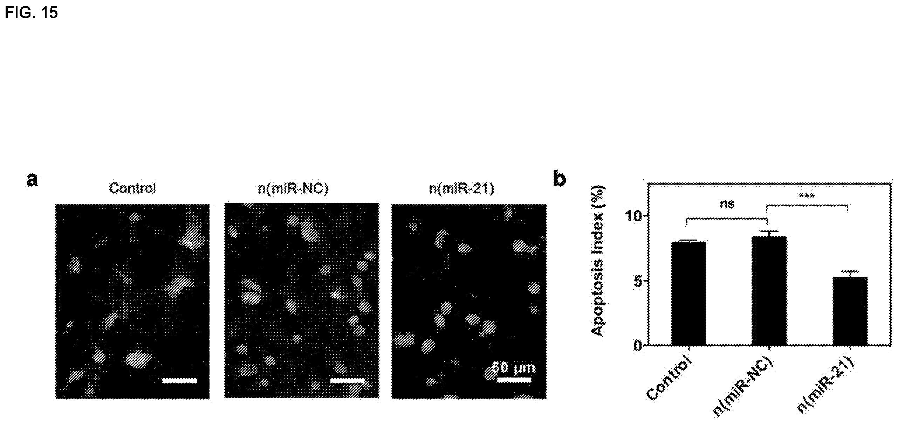

**FIG. 15** contains two panels, namely FIG. 15A and 15B, which depicts representative fluorescence images of TUNEL assay in the infarct area of brain tissues (FIG. 15A) (blue color represents DAPI and green color represents FITC) and the apoptosis indexes in the infarct area of brain tissues obtained from the quantitative analysis of TUNEL assay (FIG. 165). Data are presented as mean ± SEM (*n*=3). ****P*<0.001 (Turkey's post-test following ordinary one-way ANOVA).

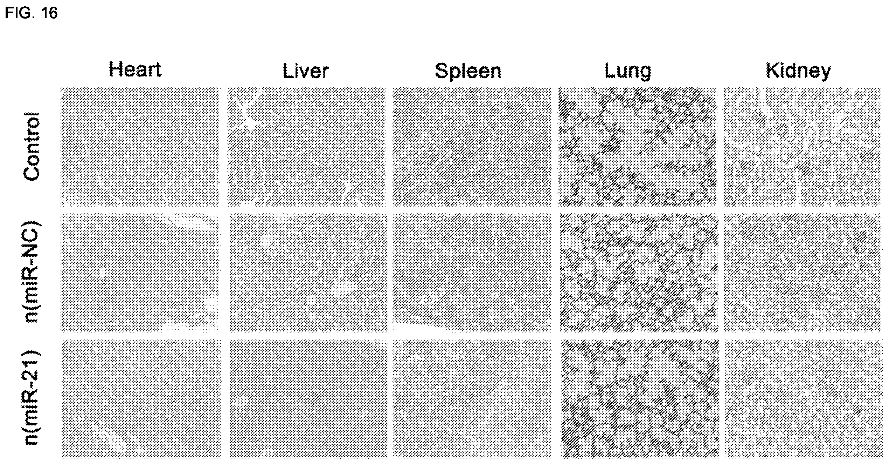

**FIG. 16** depicts representative H&E stained images of major organs after the 7-day treatment period.

### DETAILED DESCRIPTION

### Definitions

For convenience, the meaning of certain terms and phrases used in the specification, examples, and appended claims, are provided below.

As used herein, the terms "target" refers to a section of a DNA or RNA strand of a double-stranded DNA or an RNA that is complementary to a section of a DNA or RNA strand, including all transcribed regions, that serves as a matrix for transcription. A target gene, usually the sense strand, is a gene whose expression is to be selectively inhibited or silenced through RNA interference and/or microRNA.

As used herein, the term "polymer nanocapsules" refers to a composition comprising a "polymer shell" and an "RNAi molecule", such as a "microRNA molecule."

As used herein, the term "polymer shell" refers to the polymer portion of the microRNA polymer nanocapsules comprising one or more positively charged polymer monomers, one or more crosslinkers, and optionally one or more neutral polymer monomers. Examples of positively charged monomers, crosslinkers, and neutral monomers are provided in Table 1, Table 2, and Table 3 of U.S. Patent Application publication no. US 2015/0071999.

As used herein, the term "microRNA molecule" refers to any microRNA (abbreviated 'miRNA'), which is a small non-coding RNA molecule (containing about 22 nucleotides), whether found naturally (e.g., in plants, animals and some viruses), or manmade or artificially synthesized, that functions in RNA silencing and post-transcriptional regulation of gene expression (see Ambros (2004) Nature. 431:350-355; Bartel (2004) Cell 116:281-297). While naturally occurring miRNAs are typically located within the cell, some miRNAs, commonly known as circulating miRNA or extracellular miRNA, have also been found in the extracellular environment, including various biological fluids and cell culture media. A miRNA is complementary to a part of one or more messenger RNAs (mRNAs). Animal miRNAs are usually complementary to a site in the 3' UTR, whereas plant miRNAs are usually complementary to coding regions of mRNAs. Perfect or nearperfect base pairing with the target RNA promotes cleavage of the RNA. This is the typical mode of plant miRNAs. In animals, the match-ups are typically imperfect. miRNAs occasionally also cause histone modification and DNA methylation of promoter sites, which affects the expression of target genes. Nine mechanisms of miRNA action are described and assembled in a unified mathematical model: Cap-40S initiation inhibition; 60S Ribosomal unit joining inhibition; Elongation inhibition; Ribosome drop-off (premature termination); Co-translational nascent protein degradation; Sequestration in P-bodies; mRNA decay (destabilisation); mRNA cleavage; and Transcriptional inhibition through microRNA-mediated chromatin reorganization followed by gene silencing (Morozova et al. (2012) RNA 18:1635-1655).

As used herein, the terms "degradable polymer" and "nondegradable polymer" refer to the ability of the polymers described herein to degrade into smaller fragments. In certain embodiments of this invention, degradable polymers can break down at physiological pH. In certain embodiments, degradable polymers can break down at approximately pH 7.4. In certain embodiments, a mixture of degradable and nondegradable polymers can yield a degradable polymer mixture. In certain embodiments, a mixture of degradable and nondegradable polymers can break down at physiological pH. In certain embodiments, a mixture of degradable and nondegradable polymers can break down at approximately pH 7.4.

As used herein, the terms "mutant gene" and "target mutant gene" refer to a gene comprising at least one point mutation relative to the corresponding normal, non-mutated cellular gene (referred to herein as the "corresponding wild-type gene"). The terms mutant gene and target mutant gene specifically encompass any variant of a normal cellular gene or gene fragment whose expression is associated with a disease or disorder (e.g., an oncogene).

As used herein, the term "conjugate" or "conjugate agent" or "surface-conjugated targeting agent" or "polymer nanocapsule conjugates" refers to any moiety, such as a protein or effective portion thereof, that is conjugated to the polymer nanocapsules and provides specific targeting of the polymer nanocapsules to the surface of a specific cell type thereby providing directed delivery of the RNAi molecule (e.g., a microRNA) to a specific cell type. For example, the conjugate agent can bind to a cell-specific cell surface receptor, thereby bringing the polymer nanocapsules into immediate proximity to the target cell. In certain embodiments, the conjugates used to achieve specific targeting of the polymer nanocapsules include CD4, CD8, CD45, CD133, aHLA, and transferrin. In other embodiments, the conjugates can be cell-specific antibodies or fragments thereof. Additional examples of conjugates used to target specific cell types are described below in the Detailed Description.

The term "complementary RNA strand" (also referred to herein as the "antisense strand") refers to the strand of a dsRNA which is complementary to an mRNA transcript that is formed during expression of the target gene, or its processing products. As used herein, the term "complementary nucleotide sequence" refers to the region on the complementary RNA strand that is complementary to a region of an mRNA transcript of the target mutant gene (i.e., "the corresponding nucleotide sequence" of the target gene). "dsRNA" refers to a ribonucleic acid molecule having a duplex structure comprising two complementary and anti-parallel nucleic acid strands. Not all nucleotides of a dsRNA must exhibit Watson-Crick base pairs. The maximum number of base pairs is the number of nucleotides in the shortest strand of the dsRNA. The RNA strands may have the same or a different number of nucleotides. The complementary nucleotide region of a complementary RNA strand is less than 25, preferably 19 to 24, more preferably 20 to 24, even more preferably 21 to 23, and most preferably 22 or 23 nucleotides in length. The complementary RNA strand is less than 30, preferably fewer than 25, more preferably 21 to 24, and most preferably 23 nucleotides in length. dsRNAs comprising a complementary or antisense strand of this length (known as "short interfering RNA" or "siRNA") are particularly efficient in inhibiting the expression of the target mutant gene. "Introducing into" means uptake or absorption in the cell, as is understood by those skilled in the art. Absorption or uptake of nucleic acids such as microRNA can occur through cellular processes, or by auxiliary agents or devices. For example, for in vivo delivery, microRNA can be injected into a tissue site or administered systemically. In vitro delivery includes methods known in the art such as electroporation and lipofection.

As used herein, "selective inhibition of expression" means that a dsRNA and/or miRNA has a greater inhibitory effect on the expression of a target mutant gene than on the corresponding wild-type gene. Preferably, the expression level of the target mutant gene is less than 98%, less than 95%, less than 90%, less than 80%, less than 70%, less than 60%, less than 50%, less than 40%, less than 30%, less than 20%, or less than 10% of the expression level of the corresponding wild-type gene.

As used herein and as known in the art, the term "identity" is the relationship between two or more polynucleotide sequences, as determined by comparing the sequences. Identity also means the degree of sequence relatedness between polynucleotide sequences, as determined by the match between strings of such sequences. Identity can be readily calculated (see, e.g., Computation Molecular Biology, Lesk, A. M., eds., Oxford University Press, New York (1998), and Biocomputing: Informatics and Genome Projects, Smith, D. W., ed., Academic Press, New York (1993). While there exist a number of methods to measure identity between two polynucleotide sequences, the term is well known to skilled artisans (see, e.g., Sequence Analysis in Molecular Biology, von Heinje, G., Academic Press (1987); and Sequence Analysis Primer, Gribskov., M. and Devereux, J., eds., M. Stockton Press, New York (1991)). Methods commonly employed to determine identity between sequences include, for example, those disclosed in Carillo, H., and Lipman, D., SIAM J. Applied Math. (1988) 48:1073. "Substantially identical," as used herein, means there is a very high degree of homology (preferably 100% sequence identity) between the sense strand of the dsRNA and/or miRNA and the corresponding part of the target gene. However, dsRNA and/or miRNA having greater than 90% or 95% sequence identity may be used in the present invention, and thus sequence variations that might be expected due to genetic mutation, strain polymorphism, or evolutionary divergence can be tolerated. Although 100% identity is preferred, the dsRNA and/or miRNA may contain single or multiple base-pair random mismatches between the RNA and the target gene.

As used herein, the term "treatment" refers to the application or administration of a therapeutic agent to a patient, or application or administration of a therapeutic agent to an isolated tissue or cell line from a patient, who has a disorder, e.g., a disease or condition, a symptom of disease, or a predisposition toward a disease, with the purpose to cure, heal, alleviate, relieve, alter, remedy, ameliorate, improve, or affect the disease, the symptoms of disease, or the predisposition toward disease.

As used herein, a therapeutic that "prevents" a condition, disorder or disease refers to a therapeutic, such as a polymer nanocapsule (or compositions comprising them) described herein, that, in a statistical sample, reduces the occurrence of the disorder or condition in the treated sample relative to an untreated control sample, or delays the onset or reduces the severity of one or more symptoms of the disorder or condition relative to the untreated control sample.

As used herein, a "pharmaceutical composition" comprises a pharmacologically effective amount of a therapeutic agent, such as an miRNA, and a pharmaceutically acceptable carrier. As used herein, "pharmacologically effective amount," "therapeutically effective amount" or simply "effective amount" refers to that amount of an RNA effective to produce the intended pharmacological, therapeutic or preventive result. For example, if a given clinical treatment is considered effective when there is at least a 25% reduction in a measurable parameter associated with a disease or disorder, a therapeutically effective amount of a drug for the treatment of that disease or disorder is the amount necessary to effect at least a 25% reduction in that parameter.

The term "pharmaceutically acceptable carrier" refers to a carrier for administration of a therapeutic agent. Such carriers include, but are not limited to, saline, buffered saline, dextrose, water, glycerol, ethanol, and combinations thereof. The term specifically excludes cell culture medium. For drugs administered orally, pharmaceutically acceptable carriers include, but are not limited to pharmaceutically acceptable excipients such as inert diluents, disintegrating agents, binding agents, lubricating agents, sweetening agents, flavoring agents, coloring agents and preservatives. Suitable inert diluents include sodium and calcium carbonate, sodium and calcium phosphate, and lactose, while corn starch and alginic acid are suitable disintegrating agents. Binding agents may include starch and gelatin, while the lubricating agent, if present, will generally be magnesium stearate, stearic acid or talc. If desired, the tablets may be coated with a material such as glyceryl monostearate or glyceryl distearate, to delay absorption in the gastrointestinal tract.

The term "brain ischemia" refers to cerebral ischemia and/or cerebrovascular ischemia, a condition in which there is insufficient blood flow to the brain to meet metabolic demand. This leads to poor oxygen supply or cerebral hypoxia and thus to the death of brain tissue or cerebral infarction/ischemic stroke. It is a sub-type of stroke along with subarachnoid hemorrhage and intracerebral hemorrhage. Ischemia leads to alterations in brain metabolism, reduction in metabolic rates, and energy crisis. The broad term "stroke" can be divided into three categories: brain ischemia, subarachnoid hemorrhage and intracerebral hemorrhage. Brain ischemia can be further subdivided, by cause, into thrombotic, embolic, and hypoperfusion. Thrombotic and embolic are generally focal or multifocal in nature while hypoperfusion affects the brain globally. Focal brain ischemia occurs when a blood clot has occluded a cerebral vessel. Focal brain ischemia reduces blood flow to a specific brain region, increasing the risk of cell death to that particular area. It can be either caused by thrombosis or embolism. Global brain ischemia occurs when blood flow to the brain is halted or drastically reduced. This is commonly caused by cardiac arrest. If sufficient circulation is restored within a short period of time, symptoms may be transient. However, if a significant amount of time passes before restoration, brain damage may be permanent. While reperfusion may be essential to protecting as much brain tissue as possible, it may also lead to reperfusion injury. Reperfusion injury is classified as the damage that ensues after restoration of blood supply to ischemic tissue. In the instant application, the scope of brain ischemia is broad enough to cover any thrombotic, embolic, and hypoperfusion ischemia, plus any other ischemia related to and/or affecting brain functions. The main symptoms of brain ischemia involve impairments in vision, body movement, and speaking. The causes of brain ischemia vary from sickle cell anemia to congenital heart defects. Symptoms of brain ischemia can include unconsciousness, blindness, problems with coordination, and weakness in the body. Other effects that may result from brain ischemia are stroke, cardiorespiratory arrest, and irreversible brain damage. An interruption of blood flow to the brain for more than 10 seconds causes unconsciousness, and an interruption in flow for more than a few minutes generally results in irreversible brain damage.

The present invention provides a polymer nanocapsule comprising a polymer shell and a microRNA molecule for use in a method of treating cerebral or brain ischemia/reperfusion injury as defined in the claims.

This invention is based on a novel strategy through self-assembly and *in situ* polymerization technology to encapsulate microRNA molecules into cross-linked polymer nanocapsules. In certain embodiments, the polymer nanocapsules are approximately 20-100 nm in diameter. The small diameters of the polymer nanocapsules maximize the protection of the RNA molecules from external RNase attack and serum neutralization. In certain embodiments, the miRNA is selected from an miRNA that modulates genes which induce decreased apoptosis, increased angiogenesis, increased neurogenesis, or increased neuroplasticity.

In certain embodiments, the polymer nanocapsules are 10 nm-20 nm, 20-25 nm, 25 nm-30 nm, 30 nm-35 nm, 35 nm-40 nm, 40 nm-45 nm, 45 nm-50 nm, 50 nm-55 nm, 55 nm-60 nm, 60 nm-65 nm, 70-75 nm, 75 nm-80 nm, 80 nm-85 nm, 85 nm-90 nm, 90 nm-95 nm, 95 nm-100 nm, or 100 nm-110 nm. In certain embodiments, the polymer nanocapsules are approximately 10 nm, 11 nm, 12 nm, 13 nm, 14 nm, 15 nm, 16 nm, 17 nm, 18 nm, 19 nm, 20 nm, 21 nm, 22 nm, 23 nm, 24 nm, 25 nm, 26 nm, 27 nm, 28 nm, 29 nm, 30 nm, 31 nm, 32 nm, 33 nm, 34 nm, 35 nm, 36 nm, 37 nm, 38 nm, 39 nm, 40 nm, 41 nm, 42 nm, 43 nm, 44 nm, 45 nm, 46 nm, 47 nm, 48 nm, 49 nm, 50 nm, 51 nm, 52 nm, 53 nm, 54 nm, 55 nm, 56 nm, 57 nm, 58 nm, 59 nm, 60 nm, 61 nm, 62 nm, 63 nm, 64 nm, 65 nm, 66 nm, 67 nm, 68 nm, 69 nm, 70 nm, 71 nm, 72 nm, 73 nm, 74 nm, 75 nm, 76 nm, 77 nm, 78 nm, 79 nm, 80 nm, 81 nm, 82 nm, 83 nm, 84 nm, 85 nm, 86 nm, 87 nm, 88 nm, 89 nm, 90 nm, 91 nm, 92 nm, 93 nm, 94 nm, 95 nm, 96 nm, 97 nm, 98 nm, 99 nm, 100 nm, 101 nm, 102 nm, 103 nm, 104 nm, 105 nm, 106 nm, 107 nm, 108 nm, 109 nm, 110 nm, 111 nm, 112 nm, 113 nm, 114 nm, 115 nm, 116 nm, 117 nm, 118 nm, 119 nm, or 120 nm in diameter. In certain embodiments, the polymer nanocapsules are 120 nm-130 nm, 130 nm-140 nm, 140 nm-150 nm, 150 nm-160 nm, 160 nm-170 nm, 170 nm-180 nm, 180 nm-190 nm, 190 nm-200 nm, 200 nm-210 nm, 220 nm-230 nm, 230 nm-240 nm, 240 nm-250 nm, or larger than 250 nm in diameter.

In certain embodiments, polymer nanocapsules disclosed herein include nontargeting and targeting ability, higher efficiency, and/or lower adverse immune response. For example, the higher efficiency may result from increased uptake and more directed delivery.

Furthermore, highly stabilized microRNA molecules inside the protective nanocapsule are able to be fully released once the nanostructured polymer shell is degraded in endosomes and lysosomes. In certain embodiments, a nontargeting polymer encapsulated microRNA molecules can be transduced into primary cells such as PBMCs *in vitro* with superior efficiency and lower cytoxicity compared to the low efficiency and high toxicity resulting from liposome transduction.

Importantly, by choosing and designing appropriate polymer charge, the method of microRNA molecule delivery to specific purposes (such as targeting by conjugating moieties to the polymer nanocapsules as described herein) can be modulated.

In certain embodiments, the ratio of degradable crosslinker to non-degradable crosslinker is 1:1, 1:2, 2:1, 1:3, 2:3, 3:1, 3:2, 4:1, 1:4, 4:3, 3:4, 5:1, 1:5, 2:5, 5:2, 5:3, 3:5, 4:5, 5:4, 6:1, 1:6, 1:7, 7:1, 2:7, 7:2, 3:7, 7:3, 4:7, 7:4, 5:7, 7:5, 6:7, 7:6, 8:1, 1:8, 3:8, 8:3, 5:8, 8:5, 7:8, 8:7, 9:1, 1:9, 2:9, 9:2, 4:9, 9:4, 5:9, 9:5, 7:9, 9:7, 8:9, 9:8, 10:1, 1:10, 3:10, 10:3, 7:10, 10:7, 9:10, 10:9 or any other ratio that one of skill in the art would know to use. In certain embodiments, the molar ratio of the degradable cross-linker and the positively charged monomer is at least about 2:1, 1:1, 1:2, or more, preferably at least about 1:1. In certain embodiments the molar ratio of the positively charged monomer and the neutral monomer is at least about 1:1, about 1:2, about 1:3, about 1:4, about 1:5, about 1:6, about 1:7, about 1:8, about 1:9, about 1:10, about 1:11, about 1:12, about 1:13, about 1:14, about 1:15, about 1:20, or more, preferably at least about 1:10 or about 1:11.

In certain embodiments, the miRNA is selected from an miRNA that increases the expression of at least one of VEGF, GFAP, CD31, CD34, BCL-2, NeuN, bromodeoxyuridine, nestin, PSA-NCAM, doublecortin, Pax6, GAP-43, AKT, or HIF1-α. In certain embodiments, the miRNA is selected from a miRNA that decreases the expression of Caspase 3 or PTEN.

Some exemplary degradable crosslinkers and non-degradable crosslinkers are listed in Table 2 of US 2015/0071999.

In certain embodiments, the polymer nanocapsules are designed to degrade in 1 hour, or 2 hours, or 3 hours, or 4 hours, or 5 hours, or 6 hours, or 7 hours, or 8 hours, or 9 hours, or 10 hours, or 11 hours, or 12 hours, or 13 hours, or 14 hours, or 15 hours, or 16 hours, or 17 hours, or 18 hours, or 19 hours, or 20 hours, or 21 hours, or 22 hours, or 23 hours, or 1 day, or 2 days, or 3 days, or 4 days or 5 days, or 6 days, or 1 week, or 2 weeks, or 3 weeks, or 1 month or any combination thereof. In certain embodiments, the polymer nanocapsules are designed to degrade at any of the above rates at a physiological pH. In certain embodiments, the polymer nanocapsules are designed to degrade at any of the rates above post-administration to a subject in need thereof.

In certain embodiments, microRNA molecules can be effectively delivered to specific sites in vivo. In certain embodiments, a targeting agent (i.e., a conjugate or conjugate agent) is conjugated to the polymer nanocapsule. In certain embodiments the conjugation prevents the dissociation of the targeting agent from the polymer particle. In certain embodiments, cyclodextrin and/or adamantane can be used for targeting agent conjugation.

In certain embodiments, the invention is practiced using nontargeted and targeted polymer nanocapsules microRNA molecule delivery with high efficiency and low toxicity for in vitro testing and in vivo targeting to specific tissues and organs via intravenous injection.

The enhanced stability of RNAi/miRNA molecules encapsulated by the cross-linked polymer also ensures its long-lasting circulation in body before it reaches the targeting sites. Overall, the delivery of microRNA molecules as described herein can provide notable efficiency, augmented stability, and minimal toxicity.

### Monomers and Cross-Linkers

Different monomers and cross-linkers can be used to encapsulate the RNAi molecules (e.g., microRNAs) by *in situ* polymerization, such as those taught in this specification and others well known in the art.

In certain embodiments, the molar ratio of the total monomer and the miRNA is at least about 1000:1, about 2000:1, about 3000:1, about 4000:1, about 5000:1, about 6000:1, about 7000:1, 8000:1, about 9000:1, about 10000:1, or more, preferably at least about 4000:1, or about 5000:1.

### Polymer Nanocapsule Conjugates

In certain embodiments, targeted delivery of microRNA molecules into cells is achieved using surface-conjugated targeting agents on optimized nanocapsules. Of particular interest, the polymer nanocapsule conjugates can be used to target immune, pulmonary, lung, optic, liver, kidney, brain, central nervous system, peripheral nervous system, cardiac, cancer, proliferative, virally or retrovirally infected, stem, skin, intestinal, and/or auditory cells. In certain embodiments, the targeting agent delivers the polymer nanocapsule to a cell type selected from endothelial cells, microglial cells, neurons, and astrocytes.

In certain embodiments, the conjugates used to achieve specific targeting of the polymer nanocapsules include CD4, CD8, CD45, CD133, aHLA, and transferrin. In certain embodiments, the conjugates used to achieve specific targeting of the polymer nanocapsules include any one or more of the cluster of differentiation or cluster of designation (CD) markers. For example, the CD markers include CDX wherein X can be any one of 1-340. As described herein, the term "CD1", for example, means all CD1 variants and subtypes. This applies to all CD markers described herein.

In certain embodiments, the conjugates used to achieve specific targeting of the polymer nanocapsules include any one or more of AFP, beta-Catenin, BMI-1, BMP-4, c-kit, CXCL12, SDF-1, CXCR4, decorin, E-Cadherin, Cadherin 1, EGFR, ErbB1, Endoglin, EpCAM, TROP-1, Fc epsilon RI A, FCER1A, L1CAM, LMO2, Nodal, Notch-1, PDGFRB, Podoplanin, PTEN, Sonic Hedgehog, STAT3, Syndecan-1, Tranferrin Receptor, and Vimentin.

In certain embodiments, the conjugates used to achieve specific targeting of the polymer nanocapsules include any one or more of ALK, AFP, B2M, Beta-hCG, BCR-ABL, BRAF, CA15-3, CA19-9, CA-125, Calcitonin, CEA (Carcinoembryonic antigen), CD20, Chromagranin A, Cytokeratin or fragments thereof, EGFR, Estrogen Receptor, Progesterone Receptor, Fibrin, Fibrinogen, HE4, HER2/neu, IgG variants, KIT, lactate dehydrogenase, Nuclear matrix protein 22, PSA, thyroglobulin, uPA, PAI-1, and Oval.

In some embodments the targeting agent is selected from is selected from a TAT (transduction domain of human immunodeficiency virus type-1 (HIV-1)) peptide, a diphtheria toxin, a tetanus toxin, Tet1, G23, a rabies virus glycoprotein (RVG) peptide, an opioid peptide, glutathione, thiamine, leptin, an angiopep, a low-density lipoprotein, insulin, melanotransferrin, and transferrin.

Any marker described herein or acceptable to one of skill in the art can be used alone, or in combination with one or more additional markers, to achieve the desired targeting of specific cells.

### Methods of Treating Diseases Caused by Expression of a Target Gene

According to the present invention, the polymer nanocapsules are for use in treating or preventing cerebral or brain ischemia/reperfusion injury.

Although not part of the invention, the polymer nanocapsules described herein can be used in a method for treating a subject having a disease or at risk of developing a disease caused by the expression of a target mutant gene. In certain embodiments, the polymer nanocapsules described herein can be used to treat or prevent one or more of cellular proliferative and/or differentiative disorders. In certain embodiments, the polymer nanocapsules can be used to treat or prevent one or more immune or immunodeficiency disorders. In certain embodiments, the polymer nanocapsules can be used to treat or prevent viral replication or viral infection. In certain embodiments, the polymer nanocapsules can be used to treat one or more neurological or neurodegenerative disorders. In certain embodiments, the polymer nanocapsules can be used to treat or prevent cancer.

In certain embodiments, the polymer nanocapsules can be used to treat or prevent stroke or ischemia diseases or disorders, especially brain ischemia.

In certain embodiments, the polymer nanocapsules can be used to treat or prevent advanced cancers, pachyonychia congenital, age-related macular degeneration, choroidal neovascularization, metastatic melanoma, metastatic melanoma without CNS metastases, chronic myeloid leukemia, solid tumors, advanced solid tumors, optic atrophy, non-arteric anterior ischemic optic neuropathy, pancreatic cancer, pancreatic ductal adenocarcinoma, diavetic macular edema, hypercholesterolemia, colorectal cancer with hepatic metastases, pancreatic cancer with hepatic metastases, gastric cancer with hepatic metastases, breast cancer with hepatic metastases ovarian cancer with hepatic metastases, preeclampsia, neuroblastoma, ocular hypertension, open angle glaucoma, glaucoma, ocular pain, dry eye syndrome, kidney injury, acute renal failure, delayed graft function, complications of kidney transplant, TBX3 overexpression, and diabetic retinopathy.

In certain embodiments, the polymer nanocapsules can be used to treat or prevent a viral infection. In certain embodiments, the polymer nanocapsules can be used to treat or prevent a retroviral viral infection. In certain embodiments, the polymer nanocapsules can be used to treat or prevent HIV or AIDS infection. In specific embodiments, the polymer nanocapsules can be used to suppress a retroviral viral infection. In certain embodiments, the polymer nanocapsules can be used to block, prevent, or downregulate retrovirus or virus replication. In certain embodiments, the administration of the polymer nanocapsule induces a physiological process selected from anti-apoptosis, neurogenesis, and angiogenesis.

In certain embodiments, the polymer nanocapsules described herein can be administered by intravitreal injection, parenterally, intravenously, by injection into the callus on the bottom of one foot, by oral administration, subcutaneously, or by any other mode of pharmaceutical administration.

In certain embodiments, the method comprises administering a pharmaceutical composition of the invention to the subject, such that expression of the target mutant gene is silenced or down regulated. In certain embodiments, the subject is a mammal, such as a human. Because of their high specificity, the polymer nanocapsules of the present invention can specifically target the mutant genes of diseased cells and tissues, as described herein.

In the prevention of disease, the target gene may be one which is required for initiation or maintenance of the disease, or which has been identified as being associated with a higher risk of contracting the disease. In the treatment of disease, the polymer nanocapsules can be brought into contact with the cells or tissue exhibiting the disease. As one non-limiting example, polymer nanocapsules can deliver an RNA or miRNA that is substantially identical to all or part of a mutated gene associated with cancer, or one expressed at high levels in tumor cells may be brought into contact with or introduced into a cancerous cell or tumor gene. As another non-limiting example, polymer nanocapsules can deliver an RNA or miRNA that is substantially identical to all or part of a mutated gene associated with a viral or retroviral disease. Specifically, a non-limiting example of a retroviral disease that can be treated with the polymer nanocapsules described herein is HIV.

Non-limiting examples of cellular proliferative and/or differentiative disorders include cancer, e.g., carcinoma, sarcoma, metastatic disorders or hematopoietic neoplastic disorders, e.g., leukemias. A metastatic tumor can arise from a multitude of primary tumor types, including but not limited to those of prostate, colon, lung, breast and liver origin. As used herein, the terms "cancer," "hyperproliferative," and "neoplastic" refer to cells having the capacity for autonomous growth, i.e., an abnormal state of condition characterized by rapidly proliferating cell growth. These terms are meant to include all types of cancerous growths or oncogenic processes, metastatic tissues or malignantly transformed cells, tissues, or organs, irrespective of histopathologic type or stage of invasiveness. Proliferative disorders also include hematopoietic neoplastic disorders, including diseases involving hyperplastic/neoplatic cells of hematopoietic origin, e.g., arising from myeloid, lymphoid or erythroid lineages, or precursor cells thereof.

Mutations in cellular genes that directly or indirectly control cell growth and differentiation are considered to be the main cause of cancer. There are approximately thirty families of genes, called oncogenes, which are implicated in human tumor formation. Members of one such family, the RAS gene family, are carried in a broad range of eukaryotes and are frequently found to be mutated in human tumors. Polymer nanocapsules of this invention can be used to target such oncogenes to knock down or prevent their expression.

In addition to oncogenes, the methods and compositions of the invention can be applied to other disease-related target genes having a point mutation. Gene mutations have been reported in more than 1000 different human genes. Data on these mutations and their associated phenotypes have been collated and are available online through two major databases: Online Mendelian Inheritance in Man in Baltimore and the Human Gene Mutation Database in Cardiff. For example, there is a high frequency of CG to TG or CA mutations in the human genome due to deamination of 5' methyl-cytosine. Short deletions or insertions of less than 20 nucleotides are also very common mutations in humans. See, e.g., Antonarakis, S. E., Eur. Pediatr. (2000) 159(3):5173-8.

Furthermore, Sachidanandam et al. describes a map of human genome sequence variation containing 1.42 million single nucleotide polymorphisms, which is useful for identifying biomedically important genes for diagnosis and therapy (Sachidanandam, R., et al., Nature (2001) 409(6822):821-2 and Nature (2001) 409(6822):822-3). The map integrates all publicly available SNPs with described genes and other genomic features. An estimated 60,000 SNPs fall within exon (coding and untranslated regions), and 85% of exons are within 5 kb of the nearest SNP. Clifford et al. provides expression-based genetic/physical maps of single-nucleotide polymorphisms identified by the cancer genome anatomy project (Clifford, R., et al., Genome Res (2000) 10(8):1259-65). In addition to SNP maps, Sachidanandam et al. provide maps containing SNPs in genes expressed in breast, colon, kidney, liver, lung, or prostate tissue.

Accordingly, microRNA molecule polymer nanocapsules for use of this invention can be used to target such mutant genes to knock down or prevent their expression.

In some embodiments, the method of treatment comprises administering an additional therapy. In some embodiments, the additional therapy is selected from anticoagulant therapy, antiplatelet therapy or thrombolytic therapy.

### Methods of Inhibiting Expression of a Mutant Gene

According to the present invention, the polymer nanocapsules are for use in treating or preventing cerebral or brain ischemia/reperfusion injury wherein the polymer nanocapsule is administered systemically. In one embodiment, the miRNA knocks down or decreases expression of an overexpressed gene.

Although not part of the invention, the polymer nanocapsules described herein can be used in a method for inhibiting the expression of a mutant gene in subject. The method comprises administering a composition of the invention to the subject such that expression of the mutant gene is silenced as compared to the corresponding wild-type gene. In certain embodiments the subject is a mammal. In certain embodiments the mammal is a human.

Because of their high specificity, miRNA nanocapsules for use of the present invention can specifically target RNAs (primary or processed) of target mutant genes, and at surprisingly low dosages. Compositions and methods for inhibiting the expression of a target gene using polymer nanocapsules can be performed as described herein.

A composition comprising polymer nanoparticles can be administered wherein the polymer nanocapsules comprise a nucleotide sequence, such as a miRNA targeting a mutant gene. When the subject to be treated is a mammal, such as a human, the composition may be administered by any means known in the art including, but not limited to oral or parenteral routes, including intravenous, intramuscular, intraperitoneal, subcutaneous, transdermal, airway (aerosol), rectal, vaginal and topical (including buccal and sublingual) administration. In certain preferred embodiments, the compositions are administered by intravenous or intraparenteral infusion or injection.

The methods for inhibition the expression of a target gene can be applied to any mutant gene one wishes to silence, thereby selectively inhibiting its expression. Non-limiting examples of human genes which can be targeted for silencing include oncogenes cytokinin gene, idiotype protein genes, prion genes, genes that expresses molecules that induce angiogenesis, genes that encode adhesion molecules, genes that encode cell surface receptors, genes of proteins that are involved in metastasizing and/or invasive processes, genes of proteases as well as of molecules that regulate apoptosis and the cell cycle, genes that express the EGF receptor, genes that encode the multi-drug resistance 1 gene (MDR1 gene), genes that allow viral uptake and replication, genes that cause neurodegenerative disorders, genes that cause protein aggregation and/or accumulation, genes that cause upregulation or down regulation of hormones, genes that cause neurological disorders, genes that cause cardiac disorders, and genes that cause psychological disorders. One of skill in the art would understand which genes are encompassed by the broad categories of exemplary genes described above.

### Methods of Manufacture

In certain embodiments, the polymer nanocapsules described herein are manufactured to achieve a specific size, to target a specific site for gene downregulation, and to downregulate a specific gene. The size of the polymer nanocapsules described herein can be determined based on the polymer:crosslinker ratio as described herein. Targeted delivery can be achieved, for example, using conjugate agents that are attached (i.e., conjugated) to the exterior of the polymer nanocapsules as described herein. Furthermore, the specific binding of an RNAi molecule of a polymer nanocapsules described herein to a specific gene (thereby decreasing specific gene expression) can be achieved by designing the RNAi molecule using methods known to one of skill in the art. See, e.g., Birmingham et al., "A protocol for designing siRNAs with high functionality and specificity," Nature Protocols, 2007; 2(9):2068-78. Furthermore, the ability of the polymer nanocapsules to deliver the RNAi molecules to the target site can be optimized and determined by adjusting the ratios of degradable:nondegradable polymers as described herein.

### Methods of Administration

According to the present invention, the polymer nanocapsules are administered systemically.

Although not part of the invention, the pharmaceutical compositions for use in the invention may be administered by any means known in the art including, but not limited to oral or parenteral routes, including intravenous, intramuscular, intraperitoneal, subcutaneous, transdermal, airway (aerosol), rectal, vaginal and topical (including buccal and sublingual) administration. In certain preferred embodiments, the pharmaceutical compositions are administered by intravenous or intraparenteral infusion or injection.

The term "subject" to which administration is contemplated includes, but is not limited to, humans (i.e., a male or female of any age group, e.g., a pediatric subject (e.g., infant, child, adolescent) or adult subject (e.g., young adult, middle-aged adult or senior adult)) and/or other primates (e.g., cynomolgus monkeys, rhesus monkeys); mammals, including commercially relevant mammals such as cattle, pigs, horses, sheep, goats, cats, and/or dogs; and/or birds, including commercially relevant birds such as chickens, ducks, geese, quail, and/or turkeys. Preferred subjects are humans.

In some embodiments, the disclosed compositions are nasally administered in the range of once per day, to once per week, to once per two weeks, to once per month. In preferred embodiments, the microparticles and compositions are administered nasally. As used herein, the term "nasally" or "nasal administration" refers to a delivery of the microparticles to the mucosa of the subject's nose such that the microparticle content is absorbed directly into the nasal tissue.

In certain embodiments, the polymer nanocapsules, microparticles and compositions comprising them are for systemic administration.

In certain embodiments, the administration of the polymer nanocapsule increases the expression of at least one of VEGF, GFAP, CD31, CD34, BCL-2, NeuN, doublecortin, nestin, PSA-NCAM, doublecortin, Pax6, GAP-43, AKT, or HIF1-α. In certain embodiments, the administration of the polymer nanocapsule decreases the expression of at least one of Caspase 3 or PTEN.

### EXAMPLES

### Example 1: Materials and methods used in Example 2

### 1. Materials

All chemicals were purchased from Sigma-Aldrich (St. Louis, MO) and used as received without further purification unless otherwise noted. N-(3-Aminopropyl) methacrylamide hydrochloride (APM) was purchased from Polysciences Inc (Washington, PA). DiI, DAPI, Hoechst 33342, Lipofectamine 2000 and Trizol were purchased from Invitrogen. Polyvinylidene fluoride (PVDF) membrane was purchased from MilLipore, Inc. Blood chemistry assay ELISA kit, ABC-peroxidase and diaminobenzidine (DAB) and miRNA In Situ Hybridization kit were purchased from Boster Biotechnology, Inc. RNA oligonucleotides with sequences described in Table 1, FITC-labeled miR-21, Cy5.5-labeled miR-21 and Hairpin-it miRNA qPCR Quantitation Kit were ordered from GenePharma, Inc.

### 2. Instruments

UV-Visible spectra were acquired with a DU730 spectrometer (Beckman Coulter, Inc.). Dynamic light scattering (DLS) studies of the nanocapsules were performed on Zetasizer Nano instrument (Malvern Instruments Ltd., United Kingdom) equipped with a 10 mW helium-neon laser (λ = 632.8 nm) and thermoelectric temperature controller. Transmission electron microscope (TEM) images were obtained on an H-600 transmission electron microscope (Hitachi, Tokyo) with an acceleration voltage of 120 kV. Fluorescence intensities were measured with a Synergy 2 Multi-Mode Microplate Reader (BioTek, USA). Cells were observed and photographed with a Carl Zeiss Axio Observer inverted fluorescence microscope. Flow cytometry analysis was achieved using a BD LSRFortessa cell analyzer. *In vivo* near-optical fluorescent images were accessed using a CRI in vivo imaging system (Maestro, USA).

### 3. Synthesis of miRNA nanocapsules and Lipofactamine/miRNA complexes

Acrylamide (AAM), N-(3-Aminopropyl) methacrylamide (APM) and poly(ethylene glycol) methyl ether acrylate with an average Mn of 2000 Da (mPEG) were prepared as 10% (w/v) stock solution in deoxygenated RNase-free water, and glycerol dimethacrylate (GDMA) was prepared as 10% (w/v) stock solution in anhydrous DMSO. Then specific amounts of above monomers and crosslinkers were added into the miRNA solution, and the molar ratio of AAM/APM/mPEG/GDMA can be tuned for screening of the synthetic parameters. Polymerization was initiated by the addition of ammonium persulfate (1/10 molar ratio of total monomers) and N,N,N',N'-tetramethylethylenediamine (2-fold weight ratio of APS) and kept at 4 °C for 2 hr. The final miRNA concentration was tuned to 5 µM by diluting with deoxygenated RNase-free water. After polymerization, the solution was dialyzed against 10 mM PBS using a 10 kDa membrane to remove unreacted monomers and by-products. Non-PEG n(miR-21) was synthesized by a similar protocol without the use of mPEG. Agarose gel electrophoresis assay was used to observe the retardation of miRNA or nanocapsules with a UV gel image system (G:BOX F3, Syngene, UK). The particle size and zeta potential of nanocapsules were determined by Zetasizer (Nano ZS, Malvern, UK). Transmission electron microscopy (Hitachi, Tokyo, Japan) was used to observe the morphology of nanocapsules.

Lipofectamine/miRNA complexes (Lipo/miRNA) were prepared according to the manufacture's protocol. Briefly, equal volumes of Lipofectamine (1mg/mL) and miR-21 (0.16 µg/µL) were mixed thoroughly and incubated for 15 min at 4 °C for later use.

### 4. Characterizing of miRNA nanocapsules

### 4.1. Agarose gel electrophoresis assay

The gel retardation assay was carried out in 2% (w/v) agarose gel in 1 × TAE buffer at a constant voltage of 80V for 20 min. Then, the agarose gel was stained with the 0.5 mg/ml ethidium bromide solution for 30 min. Finally, the miRNA bands were visualized at 365 nm using a UV gel image system (SIM135A, SIMON).

### 4.2. Transmission electron microscopy (TEM)

TEM samples were prepared by drop coating of 5 µL of nanocapsule onto carboncoated copper grids. After 5 min, excess amount of samples was removed. The grid was then rinsed, and stained with 1% sodium phosphotungstate at pH 7.0. The grid was observed with an H-600 transmission electron microscope (Hitachi, Tokyo).

### 4.3. Dynamic light scattering (DLS)

DLS experiments were performed with a Zetasizer Nano instrument (Malvern Instruments Ltd., United Kingdom) equipped with a 10-mW helium-neon laser (λ = 632.8 nm) and thermoelectric temperature controller. The measurements were taken at 25°C with a 90° scattering angle. The sizes and the standard derivations of n(miR-21) were obtained by averaging the values of at least three measurements.

### 4.4. Zeta potential measurements

Zeta potentials of n(miR-21) were determined by photon correlation spectroscopy using a Zetasizer Nano instrument, (Malvern Instruments, Malvern, Worcestershire, UK). The measurements were performed at 25°C with a detection angle of 90°, and the raw data were subsequently correlated to Z average mean size using a cumulative analysis by the Zetasizer software package.

### 5. Impact of mPEG chain density on size distribution, zeta potential and delivery performance of nanocapsules

To understand the impact of mPEG density on size distribution, zeta potential and delivery performance of nanocapsules, nanocapsules were synthesized at a M:R ratio of 6000:1 with various mPEG/miRNA molar ratios ranged from 50 to 500 (Figure 4A). DLS was used to measure the sizes and zeta potentials of these nanocapsules, and fluorescence-activated cell sorting (FACS) was used to evaluate their internalization efficiency in model glioma cells (C6 cells, rat glioma cells) and low non-specific phagocytosis by model macrophages (J774A.1, mouse macrophages).

### 6. Stability studies of n(miR-21)

### 6.1. Stability of n(miR-21) under a physiological ionic strength

To ensure the *in vivo* stability of n(miR-21), it is critical to examine the size variation of them under a physiological ionic strength. real-time DLS measurements were employed to monitor the size variation of n(miR-21) at different times after their dialysis in PBS solution. The n(miR-21) sizes were recorded for 168 hours.

### 6.2. Stability of n(miR-21) under different temperatures

To understand the thermal stability of the n(miR-21), real-time DLS measurements were employed to monitor the size variation of n(miR-21) in PBS at 25, 37 and 60 °C. In each case, the samples were equilibrated under a given temperature for 15 min prior to data acquisitions.

### 6.3. Stability of n(miR-21) in 10% serum.

To understand the stability of n(miR-21) in presence of serum, DLS measurements were employed to observe the size variation of n(miR-21) in the mixture of serum and PBS (1:9, V/V). In each case, the samples were kept at room temperature for 168 h prior to data acquisitions (Supplementary Fig.3c).

### 6.4. Heparin stability

To understand the stability of n(miR-21) in presence of heparin, n(miR-21) and Lipo/miR-21 were incubated with 10 mg/mL heparin for 15 min at room temperature. Agarose gel electrophoresis was then carried out on 2% agarose gel and imaged by a UV gel image system (G:BOX F3, Syngene, UK).

### 6.5. Serum stability and RNase stability of encapsulated miR-21

To understand the stability of n(miR-21) in the presence of serum, real-time PCR was carried out to measure the relative quantification of residual miR-21 before and after miR-21 formulations treatment with serum (Shi et al. (2013) Angew Chem Int Ed Engl 52:3901-3905). Native miR-21, n(miR-21) and Lipo/miR-21 containing 1µg miR-21 were incubated in 50% fetal bovine serum for 1 hour at 37 °C, respectively. Then the miRNAs were extracted by chloroform/ 0.1% SDS-0.5 M NaCl and reconstituted to 100 µL with RNase free water, respectively (Yan et al. (2012) J Am Chem Soc 134:13542-13545). cDNA was synthesized using the Hairpin-it miRNA qPCR Quantitation Kit (GenePharma). Relative quantification of residual miR-21 level was conducted using amplification efficiencies derived from cDNA standard curves. Data were analyzed by DNA Engine Opticon 2 Two-Color Real-time PCR Detection System (Bio-Rad) and normalized to the expression of untreated native miR-21 (fold change, 2^{-δCT}). All RT-PCR reactions were performed in triplicate. For the nuclease stability study, native miR-21, n(miR-21) and Lipo/miR-21 containing 1 µg miR-21 were incubated in 10 mM Tris-HCl (pH 8.0), 10 mM EDTA for 1 hour at 37 °C in the presence of 1 mg/mL RNase A. Then, miRNAs were extracted by chloroform/ 0.1% SDS-0.5 M NaCl and reconstituted to 100 µL with RNase free water (Yan *et al.* (2012), *supra).* Relative quantification of miR-21 level was detected by the method described in the serum stability study.

### 7. Protein adsorption assay

Protein adsorption was quantified using the BCA Protein Assay (Thermo Scientific) (Liang et al. (2016) Nano Research 9:1022-1031). Briefly, 10 µL of PBS (negative control), non-PEG n(miR-21), n(miR-21) and Lipo/miR-21 were mixed with 30 µL of mouse whole serum and incubated at 37 °C for 30 min, respectively. After incubation, samples were filtered and washed with PBS for 3 times with centrifugal filtration (molecular weight cut-off, MWCO = 100 kDa) to remove any unabsorbed serum proteins. After reconstituting with 50 µL of PBS, the overall protein concentration of each sample was measured using a BCA Protein Assay according to the manufacturer's instructions. Samples were reacted with working reagent at 37 °C for 30 min, and absorbance was then measured at 562 nm. Values were compared with a bovine serum albumin standard curve. The amount of protein adsorbed was calculated using the overall protein concentration and unabsorbed serum proteins concentration.

### 8. Degradation kinetics and miRNA release study

Degradation of miRNA nanocapsules was investigated in 50 mM sodium acetate buffer (pH 5.4) and 50mM PBS buffer (pH 7.4). Degradation kinetics of the nanocapsules was quantified using the scattering light intensity ratio *I*/*I*₀ by DLS technique, where *I*₀ represents the initial nanocapsules scattering light intensity and *I* represents the timedependent nanocapsules scattering light intensity (Liu et al. (2015) Adv Mater 27:292-297; Gu et al. (2009) Nano Lett 9:4533-4538). Then, the release of miR-21 from n(miR-21) was investigated by agarose gel electrophoresis assay after their incubation with or without 1 mg/mL heparin, then imaged by a UV gel image system.

### 9. In vitro studies

### 9.1. Cell lines and Cell culture.

Authenticated J774A.1 cells and Glioma C6 cells were obtained from American Type Culture Collection (ATCC, Rockville, MD), tested for mycoplasma contamination and cultured in Dulbecco's modified Eagle's medium (DMEM, Gibco) supplemented with 10% fetal bovine serum (FBS, Invitrogen), 4 mM glutamine, 100 U/ml penicillin and 100 µg/ml streptomycin in an incubator (Thermo Scientific) at 37 °C under an atmosphere of 5% CO₂ and 90% relative humidity. All the cells used in this study were in the logarithmic phase of growth.

### 9.2. Cell viability assay.

The toxicity of the nanocapsules was assessed by MTT assay using native miR-NC as control. C6 cells (2000 cells/well) were seeded on a 96-well plate the day before exposure to nanocapsules. miR-NC nanocapsules, Lipo/ miR-NC complexes and miR-NC with various miRNA concentrations were incubated with cells for 4 hours, removed from the mixture, and incubated with fresh media for another 48 hours. The MTT solution (20 µL) was added to each well and incubated for 4 hr. The medium was then removed and 150 µL DMSO was added into each well. The plate was placed on a shaking table, 150 rpm for 5 min to thoroughly mix the solution, and then absorbance readings were measured at 560 nm using a Synergy 2 Multi-Mode Microplate Reader (BioTek, USA). Untreated cells were used as the 100% cell viability control.

### 9.3. Intracellular trafficking and phagocytosis studies.

C6 (2 × 10⁵ cells) were seeded into a 6-well plate containing coverslips in the wells and cultured for attachment, respectively. FITC-labeled native miR-21 and n(miR-21) were added into wells at a final concentration of 50nM miRNAs for 4 h incubation at 37 °C. Cells were then stained by 5 µg DiI according to the manufacturer's protocol. Cells were fixed with 4% formaldehyde in PBS for 20 min at room temperature, then washed by PBS for three times. Lastly, coverslips were placed onto glass slides with 20 µL aqueous mounting medium. The slides were then observed using FluoView Confocal Laser Scanning Microscopes-FV1000 (Olympus, Tokyo, Japan)

The cellular uptake efficiency of n(miR-21) was assayed by fluorescence-activated cell sorting (FACS). C6 cells were seeded at a density of 2 × 10⁵ cells/well into a 6-well plate and cultured for attachment. Then, 50 nM FITC-labeled native miR-21, Lipofectamine complexed miR-21 (denoted as Lipo/miR-21) and n(miR-21) were transduced at 37 °C for 4 hr. After collecting cells in wells by trypsin digestion and centrifugation, cells were washed twice by PBS buffer containing 1%(w/v) heparin, then resuspended in 0.3 mL PBS buffer containing 40 µg/ml trypan blue for flow cytometer analysis (EPICS XL, Beckman Coulter).

To evaluate phagocytic activity, 1 × 10⁵ J774A.1 cells were seeded on 6-well plate and allowed to attach and grow. After 48 hours, cells were incubated with n(miR-21), non-PEG n(miR-21) and Lipo/miR-21 at a dosage of 50 nM miRNA, then incubated for another 2 hours for phagocytosis. The cells were washed twice with PBS labelled with Hoechst 33342 for 10 minutes and analyzed using a Carl Zeiss Axio Observer inverted fluorescence microscope. Further quantification of fluorescence intensities was assayed by FACS with a similar protocol as the C6 cells.

### 9.4. Quantitative real-time polymerase chain reaction (qRT-PCR) assay.

Quantitative real-time polymerase chain reaction (qRT-PCR) was used to quantitatively compare the miRNA regulation ability of nanocapsules versus Lipofectamine. C6 cells were seeded into 6-well plates at a density of 2 × 10⁵ cells/well for 24 hours. Then, cells were treated with miRNA nanocapsules or miRNA Lipofectamine complex with a final concentration of 50 nM for 4 hr at 37 °C in serum-free medium or 50% human serum medium. Mediums were later changed to DMEM with 10% Bovine Fetal Serum. After 24 hours, total RNA was isolated from cultured cells with Trizol according to the manufacturer's instructions. To detect miR-21, stem-loop reverse transcription-polymerase chain reaction (RT-PCR) was performed with a Hairpin-it miRNA qPCR Quantitation Kit (GenePharma) according to the manufacturer's instructions. Real-time PCR was carried out by SYBR green detection with a forward primer for the mature miRNA sequence and a universal adaptor reverse primer (miR-21-R: GTCGTATCCAGTGCAGGGTCCGAGGTATTCGCACTGGATACGACtcaaca (SEQ ID NO:1); and miR-21- F: GCCGCTAGCTTATCAGACTGATGT) (SEQ ID NO:2) (Liu *et al.* (2015), *supra*)*.* The relative expression of miR-21 was evaluated by the comparative CT (threshold cycle) method and was normalized to the expression of U6 small RNA. Primers for miR-21 and U6 were obtained from GenePharma (Shanghai, China). All RT-PCR reactions were performed in triplicate.

### 9.5. Western blotting analysis.

In the western blotting analysis, C6 cells were transduced with n(miRNA) for 48 hours. Total protein yield was quantified using NanoDrop 2000 (Thermo Scientific). The protein lysates were separated by SDS-PAGE, then transferred to polyvinylidene fluoride (PVDF) membranes (MilLipore, Billerica, MA, USA). The membranes were incubated with primary antibodies against PTEN (1: 1000, Santa Cruz Biotechnology, #sc-7974), AKT (1:1000, Santa Cruz Biotechnology, #sc-81434), HIF 1-α (1:1000, Cell Signaling Technology, #14179), and VEGF (1: 1000, Santa Cruz Biotechnology, #sc-507), followed by incubation with an HRP-conjugated secondary antibody (1:5000, Promega Corporation). GAPDH (1:1000, Santa Cruz Biotechnology, #sc-47724) was set as a loading control.

### 10. In vivo studies.

### 10.1. In vivo imaging study.

Cy5.5 labeled Lipo/miR-21 or n(miR-21) injected into the tail vein of animals (n=3) at a dose of 0.5mg miRNA/kg rat after induction of ischemia. Equal volume of PBS was administrated to the control rats. Then, 20 µL of blood was collected from the tail vein at 0.08333, 0.25, 0.5, 1, 2, 4, 12 and 24 hours after injection and subsequently diluted with 30 µL PBS in a 96-well microplate before fluorescence measurement. The Cy5.5 fluorescence intensity measurements were performed on a VARIAN Cary Eclipse fluorescence spectrofluorometer, using an excitation wavelength of 678 nm with emission recorded at the wavelength range of 690-750 nm and a scan rate of 125 nm min⁻¹. After collecting the blood, rats were anesthetized and imaged by CRI *in vivo* imaging system (Maestro, USA). Then, the animals were sacrificed and organs including liver, spleen, heart, lung, kidney, and brain, were excised for near-optical fluorescent imaging to check the accumulation of fluorescent nanoparticles. The intensity of imaging signals from regions of interest (ROIs) were selected, measured, and analyzed in total photons/second and maximum photons/second/cm²/steradian using LIVING IMAGE Optical Imaging Software (Maestro). After *in vivo* imaging analysis, the excised brains were frozen in liquid nitrogen and then ground finely. Total RNA was isolated with Trizol according to the manufacturer's instructions, followed by quantitative measurement of miR-21 expression levels in the brain tissues using the qRT-PCR method.

### 10.2. Delivery efficiency comparison between the intracarotid and intravenous administration routes

To better estimate the delivery efficiency of n(miR-21) to ischemic brain after systemic delivery, Cy5.5 labeled n(miR-21) were administrated to animals (n=3) through intracarotid (*i.c.*) injection at a dose of 0.1mg miRNA/kg rat, intravenous (*i.v.*) injection at a dose of 0.5mg miRNA/kg rat, or 0.1mg miRNA/kg rat one-day post creation of ischemia. 24 hours after injection, rats were anesthetized and imaged by CRI *in vivo* imaging system (Maestro, USA). Then, the animals were sacrificed and brain tissues were excised for near-optical fluorescent imaging to check the accumulation of fluorescent nanoparticles. qRT-PCR assay was also carried out to detect the miR-21 levels in the excised brains after the *in vivo* imaging.

### 10.3. Blood chemistry assay

Liver function was evaluated by measuring the serum enzymatic parameters. Briefly, 24h after samples injected, 2.0 mL blood samples were taken from canthus vein with the capillary tube, respectively. The blood samples were centrifuged by 300r/min at room temperature, and then upper serum was collected to perform the serum biochemical assay using the ELISA kits. The test includes examining the level of various enzymes like aspartate aminotransferase (AST), alanine aminotrasferase (ALT), alkaline phosphatase (ALP), total protein and albumin (TP).

### 10.4. miR-21 detection by fluorescence in situ hybridization.

Qualitative miR-21 expression in brain tissues was detected by fluorescence *in situ* hybridization with an *in situ* hybridization kit (Boster, Wuhan, China) according to the manufacturer's protocol. Cy3-avidin was used to label miR-21 at a concentration of 0.5 mg/mL. Nuclei were counterstained with DAPI and visualized by a Carl Zeiss Axio Observer inverted fluorescence microscope.

### 10.5. Histology and immunohistochemistry.

The paraffin-embedded tissue sections were used for HE staining and the examination of VEGF (1:100, Santa Cruz Biotechnology, #sc-507), CD31 (1:100, Santa Cruz Biotechnology, #sc-1506), HIF 1-α (1:100, Cell Signaling Technology, #14179) and GFAP (1:100, Cell Signaling Technology, #3670) expression. Sections were incubated with primary antibodies overnight at 4°C, followed by biotin-labeled secondary antibody (1:100, Zhongshan Bio Corp) for 1 hour at 37°C, then incubated with ABC-peroxidase and diamino-benzidine (DAB) (1:100, Zhongshan Bio Corp, #SP-9000-D), counterstained with hematoxylin, and lastly visualized using light microscopy.

### 10.6. Apoptosis assay

The paraffin-embedded tissue sections were for the examination of cell apoptosis in the IBD. TUNEL assay was performed using an *In Situ* Cell Death Detection Kit (Roche) according to the manufacturer's protocol. Then the sections were imaged by a Carl Zeiss Axio Observer inverted fluorescence microscope. Quantitative analysis was performed using ImageJ software (NIH).

### 10.7. Histopathology analysis of major organs

Histopathology analysis was performed as mentioned above. The paraffin-embedded sections were stained with hematoxylin and eosin for histopathologic evaluation. Injuries were examined microscopically for evidence of cellular damage and inflammation.

### 11. Statistical Analysis

All quantitative results were from at least 3 separate experiments performed in triplicate, unless otherwise noted. Evaluation of significance was performed by two-tailed unpaired Student's t-test (for comparisons of two groups) analysis of variance (for comparisons of more than two groups) with corrections for multiple comparisons using Graphpad Prism 6 Software. All the data meet assumptions of the statistical test and variance was calculated to be similar between groups compared. Results were considered significant if *P*<0.05 or less.

### 12. Synthesis and Characterization of miRNA nanocapsules

Stock solutions of acrylamide (AAM), N-(3-Aminopropyl) methacrylamide (APM) and poly(ethylene glycol) methyl ether acrylate (mPEG) (average Mn 2000) were prepared (10% w/v) in deoxygenated RNase-free water. Glycerol dimethacrylate (GDMA) was prepared as 10% (w/v) stock solution in anhydrous DMSO. Then specific amounts of above monomers and crosslinkers were added into the miRNA solution, and the molar ratio of AAM/APM/mPEG/GDMA can be tuned for screening purpose. Polymerization was initiated by adding ammonium persulfate (1/10 molar ratio of total monomers) and N,N,N',N'-tetramethylethylenediamine (2-fold weight ratio of APS) and kept at 4 °C for 2 hr. The final miRNA concentration was tuned to 5 µM by diluting with deoxygenated RNase-free water. After polymerization, the solution was dialyzed against 10 mM PBS using a 10 kDa membrane to remove unreacted monomers and by-products. Non-PEG n(miR-21) was synthesized by a similar protocol without the use of mPEG. Agarose gel electrophoresis assay was used to observe the retardation of miRNA or nanocapsules with a UV gel image system (G:BOX F3, Syngene, UK). The particle size and zeta potential of nanocapsules were determined by Zetasizer (Nano ZS, Malvern, UK). Transmission electron microscopy (Hitachi, Tokyo, Japan) was used to observe the morphology of nanocapsules. Sequences of miRNAs we used are list in Table 1.

**Table 1 Sequences of miRNAs used in this study.**

| Name | Oligonucleotides sequence |
|---|---|
| Antisense miRNA-21 (SEQ ID NO:1) | UCAACAUCAGUCUGAUAAGCUA-3' 5'- |
| Antisense miRNA negative control (SEQ ID NO:2) | 5'-CAGUACUUUUGUGUAGUACAA-3' |
| miRNA-21 mimics (SEQ ID NO:3) | 5'-UAGCUUAUCAGACUGAUGUUGA-3' |
| miRNA mimics negative control (SEQ ID NO:4) | 5'-UUCUCCGAACGUGUCACGUTT-3' |

### 13. Transient focal cerebral ischemia rat model

Male Sprague-Dawley (SD) rats, weighing 260-280 g were randomly allocated to experimental groups, and weight-matching was ensured before beginning experimental protocols. Investigators were not blinded to treatment group during studies unless otherwise noted. The transient focal cerebral ischemia rat model was induced by middle cerebral artery occlusion reperfusion (MCAO/R) as previously described (Liu et al. (2013) Biomaterials 34,817-830). Briefly, SD rats were anesthetized with 10% chloralic hydras (350 mg/kg, intraperitoneally (i.p.)). Body temperature was monitored and maintained at 37 °C. A midline incision was made on the ventral side of the neck and muscles were gently pulled aside; then, the right common carotid artery and the junction of internal and external carotid artery were dissected carefully. The external carotid artery was ligated and cauterized. A surgical nylon monofilament (diameter 0.234 mm) with its tip rounded by heating near a flame was inserted into the internal carotid artery through a nick of the external carotid stump to block the origin of middle cerebral artery. After 1 hour of ischemia, the filament was pulled out for reperfusion.

Neurological scores were assessed 24 hours after MCAO/R for the confirmation of successful building of transient focal cerebral ischemia model (Liu *et al.* (2013), *supra*)*.* An examiner blinded to the experimental groups performed behavior assessments. Neurological deficit was scored based on the following description: 0, no deficits; 1, difficulty in fully extending the contralateral forelimb; 2, unable to extend the contralateral forelimb; 3, mild circling to the contralateral side; 4, severe circling and 5, falling to the contralateral side. Rats with neurologic deficit scores ranged from 1-3 were selected as the model. After neurological scores assess, rats were lethally anesthetized, and brain slices was harvested for HE staining to confirm the successful building of transient focal cerebral ischemia model.

### 14. In vivo Therapeutic efficacy

Thirty transient focal cerebral ischemia model rats were divided into three groups at random. n(miR-NC) and n(miR-21) were injected into the tail vein of the model rats at a dose of 0.5 mg miRNA/kg rat every two days for three times; equal volume of PBS was administrated to the control rats. Neurological scores were evaluated before each injection by the method described above. At day 7, two days after the last injection, animals were sacrificed after the last neurologic deficit scores evaluation. Brains were quickly removed, coronally sectioned at 1 mm intervals, and stained by immersion in the vital dye (2%) 2,3,5-triphenyltetrazolium hydrochloride (TTC). The infarct volume was calculated by summarizing the infarction areas of all sections, and multiplying the total by slice thickness. To avoid the potential effects of edema on infarct volume value, expression of the infarct volume as percentage of the infarct was calculated by dividing the infarct volume by the total ipsilateral hemispheric volume (Pignataro et al. (2009) FEBS J 276:46-57). The infarct area was evaluated from scanned digital images using Image J software (NIH, Bethesda, MD), and represented as a percentage. Brain coronal sections were fixed in 4% paraformaldehyde, dehydrated, and subsequently embedded in paraffin for histochemistry analysis.

### Example 2. Systemic Delivery of microRNA for Treatment of Brain Ischemia

A platform technology is described herein based on microRNA nanocapsules, which enables their effective delivery to the disease sites in the brain. Exemplified by microRNA-21, intravenous injection of the nanocapsules into a rat model of cerebral ischemia could effectively ameliorate the infarct volume, neurological deficit and histopathological severity. An exemplary nano-encapsulating technique, whereby miRNA molecules are encapsulated within a thin network-polymer shell by *in situ* polymerization, is illustrated in FIG. 1A. Briefly, miRNAs are first incubated with mixed monomers and degradable crosslinker, glycerol dimethacrylate (GDMA). The mixed monomers include acrylamide (AAM), poly(ethylene glycol) methyl ether acrylate (mPEG), and positively-charged monomer N-(3-aminopropyl)methacrylamide (APM). Driven by noncovalent interactions (e.g., the electrostatic interactions between the negatively charged miRNAs and APM), the monomers and cross-linkers are enriched around the miRNA molecules (Step I). Subsequently, free-radical polymerization is initiated to form a thin polymer layer around the miRNA core, yielding miRNA nanocapsules (denoted as n(miRNA)). The GDMA molecules crosslink the polymer chains, creating a network structure around the core miRNAs (Step II). GDMA is stable at neutral pH but degradable in acidic environment (Yan et al. (2010) Nat Nanotechnol 5:48-53). The nanocapsules (the polymer shells) are stable under the physiological condition (pH 7.4); upon entering the endosomes with acidic environment (pH 5.5), the GDMA crosslinkers are cleaved and the shells are degraded, allowing the release of the miRNA cargo. As illustrated in FIG. 1B, upon intravenous injection, increased permeability of the blood vessels in ischemic tissues (Kim et al. (2011) Nano Lett 11:694-700; England et al. (2016) Biomaterials 100:101-109) allows extravasation and accumulation of the nanocapsules in the ischemic tissue, which enables delivery of n(miRNA) to the target site (I). Upon internalization of the nanocapsules by the cells, miRNA is released to the cytoplasm (II), inducing angiogenesis and regeneration of the ischemic tissue (III).

### Results

### Synthesis of the miRNA nanocapsules.

MiR-21, a miRNA exhibiting angiogenesis and anti-apoptotic activity in many diseases (Buller et al. (2010) FEBS J 277:4299-4307; Chan et al. (2005) Cancer Res 65:6029-6033), was used as demonstration for effective delivery of miRNA for brain tissue regeneration. FIG. 2A shows the gel electrophoresis of the miR-21 nanocapsules (n(miR-21)) synthesized using various monomers-to-miR-21 molar ratios (M/R) of 500:1, 1000:1, 2000:1, 4000:1, 6000:1, 8000:1, and 10000:1) with a fixed AAM:APM:mPEG:GDMA molar ratio of 54:5:1:6 (FIG. 3A). The samples prepared with M/R ratios lower than 4000:1 exhibit a native miR-21 band, suggesting incomplete encapsulation of the miR-21. When the M/R ratios are higher than 4000:1, the samples show absence of the native miR-21 band and migrate towards the anode direction, indicating complete encapsulation of miR-21 within the cationic polymer shells. This observation is in consistence with their zeta potential obtained by dynamic light scattering measurements (DLS, FIG. 3C).

To further confirm the miR-21 molecules were encapsulated within the polymer shells, Lipofectamine/miR-21 complexes (Lipo/miR-21) and n(miR-21) prepared with M/R ratios from 4000 to 10000 were incubated with 10 mg/mL heparin. Lipo/miR-21 were formed through assembling negatively-charged miR-21 with positively-charged lipofectamine via noncovalent interactions, which are unstable in the presence of charged biomolecules such as heparin. As expected, gel electrophoresis shows extraction of the miR-21 from Lipo/miR-21 upon disassembly of the complexes in the presence of heparin. In contrast, for n(miR-21), miR-21 was encapsulated within network of polymer shells and no extraction of miR-21 is observed from n(miR-21) after the heparin incubation, suggesting a highly stable encapsulating structure (FIG. 2B).

The nanocapsules synthesized with M/R ratios from 4000:1 to 10000:1 show slightly decreasing hydrodynamic diameters from ~28 nm to ~18 nm (FIG. 3B) and increasing zeta potential from ~6.9 mV to ~13.8 mV (FIG. 3C). At the fixed M/R ratio of 6000:1, n(miR-21) were also synthesized with varied mPEG/miRNA ratios (50:1, 100:1, 250:1 and 500:1 FIG. 4A and FIG. 4B), which exhibit a similar hydrodynamic diameter of ~30 nm (FIG. 4C). However, the zeta potentials decrease from ~11.2 mV to ~4.6 mV with increasing percentage of mPEG (FIG. 4D). Based on their relatively high internalization efficiency in model glioma cells (C6 cells, rat glioma cells) and low non-specific phagocytosis by model macrophages (J774A.1, mouse macrophages) (FIG. 5), the nanocapsules synthesized using the M/R ratio of 6000:1 and mPEG/miRNA ratio of 100:1 were used for further studies. Such nanocapsules are of spherical morphology with diameter of ~25 nm and surface charge of ~10 mV, which were confirmed by transmission electron microscopy (TEM) (FIG. 2C) and DLS (FIG. 2D and FIG. 2E).

### Storage stability, RNase stability, non-specific protein adsorption, and releasing kinetics of miRNA nanocapsules.

To examine their storage stability, hydrodynamic diameters of n(miR-21) was monitored by DLS measurement in different conditions: (i) different storage time in presence of physiological salt concentration at 4°C; (ii) different storage temperature (25 °C, 37 °C and 60°C); and (iii) stored with and without a presence of 10% serum. The results indicate that the nanocapsules are stable in PBS solution at 4°C for one week (FIG. 6A), while the presence of 10% serum induces aggregation of the nanocapsules during the one-week storage (FIG. 6C). Moreover, the variation of storage temperature results in no change of the n(miR-21) size (FIG. 6B).

Nanocapsules exhibit enhanced stability against ribonuclease (RNase) and minimized non-specific adsorption of proteins. After incubation with RNase A and mouse serum for 2 hr, miRNAs were extracted from both Lipo/miR-21 and n(miR-21), followed by a real-time PCR quantification41. As shown in FIG. 2F, compared to the native miR-21 and Lipo/miR-21, n(miR-21) shows more effective protection of the encapsulated miR-21 against RNase and serum. Moreover, the mPEG chains on the nanocapsules minimize non-specific adsorption of proteins. As illustrated in FIG. 2G, after incubation with mouse serum at 37°C for 30 min, n(miR-21) exhibits minimal protein adsorption, whereas Lipo/miR-21 shows significant protein adsorption. To validate that the mPEG chains on the shell of nanocapsules are responsible for the reduced protein adsorption, compared nanocapsules were synthesized without mPEG (only with AAM and APM monomers) (denoted as non-PEG n(miR-21)), which show significant serum protein adsorption (FIG. 7). The reduced non-specific adsorption of proteins is essential to prolong their circulation lifetime (Gref et al. (1995) Adv Drug Deliv Rev 16:215-233).

The degrading kinetics of n(miR-21) in an acidic environment (e.g., pH 5.5, similar to the endosomal environment) was dynamically monitored by measuring the scattering light intensity using DLS (Gu *et al.* (2009), *supra;* Liu *et al.* (2015), *supra).* As shown in FIG. 2H, under an endosome-mimicking environment (pH 5.5), degradation of n(miR-21) was evidenced by the decrease of the relative scattering light intensity from 100% to ~40% within a 2-hr incubation at 37°C, while no significant change in the relative scattering light intensity was observed at pH 7.4. Agarose gel electrophoresis was used to further confirm the release of miRNA from the nanocapsules upon their degradation. As shown in FIG. 2I, clear extraction of miR-21 is observed in the presence of 1 mg/mL heparin at pH 5.5, while no extraction of miR-21 from n(miR-21) was observed at pH 7.4.

### Cellular internalization efficiency, non-specific phagocytosis, cytotoxicity, and bioactivity of miRNA nanocapsules.

To estimate the delivery efficiency, miR-21 was firstly labeled with fluorescein isothiocyanate (FITC) and encapsulated within the nanocapsules. Cell internalization of the n(miR-21) were evaluated using C6 cells. Fluorescent images of the C6 cells after 4-hr incubation with miR-21, n(miR-21) and Lipo/miR-21 are shown in FIG. 8A. Both n(miR-21) and Lipo/miR-21 show effective internalization of miR-21 in the C6 cells. Based on the intensity of FITC quantified by Flow Cytometer, the internalization efficiency of n(miR-21) reaches ~60% in the C6 cells (FIG. 8B).

Non-specific phagocytosis of both n(miR-21) and Lipo/miR-21 was evaluated in J744A.1 mouse macrophages. Compared to Lipo/miR-21, n(miR-21) shows 10-fold lower non-specific phagocytosis (FIG. 8D, FIG. 8E, and FIG. 9). Furthermore, scrambled miRNAs (miR-NC) were used to synthesize n(miR-NC) and Lipo/miR-NC to study the cyctotoxicity. The Lipo/miR-NC shows increasing toxicity with the increase of dosage, while n(miR-NC) exhibits comparable cell viability with miR-NC itself, indicating minimal toxicity from polymer shell of n(miR-NC) (FIG. 8C).

To examine their bioactivity, miR-21 expression in C6 cells was quantitatively compared between two delivery systems through qRT-PCR. miR-NC was first delivered by both systems to monitor their disruptions of miR-21 expression. Neither of them shows obvious effect on miR-21 expression (FIG. 10). The miR-21 transfection efficiency was further compared between n(miR-21) and Lipo/miR-21. As shown in FIG. 8F, without serum, Lipo/miR-21 and n(miR-21) up-regulate the miR-21 expression to 19-fold and 12-fold, respectively. However, with the presence of serum, n(miR-21) still up-regulates the expression to more than 8-fold, while Lipo/miR-21 only up-regulates the miR-21 expression to about 5-fold, losing 75% of its capacity. Antisense miR-21 (AS-miR-21) was also used to compare the miRNA transfection ability of these two methods. In the serum-free condition, n(AS-miR-21) and Lipo/miR-21 efficiently down-regulate miR-21 expression to 26% and 18%, respectively. Moreover, in the presence of serum, n(AS-miR-21) still significantly knocks down the miR-21 expression to 32%, while Lipo/miR-21 only keeps 56% silence efficacy (FIG. 10). These results confirm that the nanocapsules-delivery system is a safe yet effective for regulating intracellular miRNA level.

To further evaluate the activity of the miR-21 released from nanocapsules, the regulation of its downstream proteins associated with angiogenesis, hypoxia and apoptosis was analyzed by western blot assay. Results show that the delivery of n(miR-21) effectively increases the expression of AKT, HIF1-α and VEGF, while decreases that of PTEN (FIG. 8G and FIG. 11A). Meanwhile, n(AS-miR-21) regulate those proteins oppositely. Furthermore, neither n(miR-NC) nor n(AS-miR-NC) disturbs the expression of any aforementioned proteins (FIG. 11B), further indicating the safety and effectiveness of such nanocapsules.

### Biodistribution, pharmacokinetics, hepatotoxicity and delivery efficacy of miRNA nanocapsules by intravenous administration.

The *in vivo* application of the miRNA nanocapsules for brain ischemia therapy was demonstrated in a rat model of transient focal cerebral ischemia induced by middle cerebral artery occlusion reperfusion (MCAO/R) (FIG. 13). MiR-21 labeled with Cy5.5 (Cy5.5-miR-21) was used to synthesize Lipo/miR-21 and n(miR-21). Then, these two samples were injected intravenously 1 day after ischemia injury26, 39 at a dosage of 0.5 mg/kg miR-21, respectively. Accumulation of the samples in both ischemic and non-ischemic brains were analyzed using both fluorescence imaging and qRT-PCR 24 hours after administration.

Both ischemic and non-ischemic rats administrated with n(miR-21) show stronger fluorescent intensity in the heads and isolated brain tissues than those with Lipo/miR-21 in (FIG. 12A). The radiant efficiency of miR-21 delivered by n(miR-21) or Lipo/miR-21 was quantitatively compared in both ischemic and non-ischemic rats. As shown in FIG. 12B, the radiant efficiency of n(miR-21) is 2-fold and 3.5-fold higher than that of Lipo/miR-21 and PBS control in the ischemic rats, respectively, indicating more efficient delivery of n(miR-21) to the ischemic brain tissue. The miR-21 expressions in both ischemic and non-ischemic brain tissues after delivery were further compared by qRT-PCR (FIG. 12C). The level of miR-21 in the brains of ischemic rats treated by n(miR-21) exhibits 2.7-fold greater in comparison with those treated by Lipo/miR-21. Insignificant variation in miR-21 level was observed in the non-ischemic brains treated with either Lipo/miR-21 or n(miR-21), indicating n(miR-21) enhances the regulation in the ischemic brain tissues in comparison with Lipo/miR-21.

Major organs including liver, spleen, kidney, heart and lung were collected for biodistribution study through *ex vivo* imaging. Minimal signal from n(miR-21) could be detected in the organs related to the reticuloendothelial system (RES) (e.g., liver and spleen), while obvious accumulation of Lipo/miR-21 was observed in the RES organs (FIG. 12D). Further quantified radiant efficiency of the organs show 5-fold, 3-fold, and 3.5-fold higher of Lipo/miR-21 accumulation in liver, spleen and kidney than that of n(miR-21), respectively (FIG. 12E). The pharmacokinetic profiles of Lipo/miR-21 and n(miR-21) were also evaluated by quantitatively detecting the Cy5.5 intensity in the plasma. As expected, n(miR-21) shows enhanced blood persistence compared with Lipo/miR-21 (FIG. 12F). The concentration-time curves were fit using a two-compartment model, and the resulted PK parameters are listed in Table 2. The area under the concentration-time curves, elimination half-life (t_{1/2}), and mean residence time of n(miR-21) are remarkably higher than those of Lipo/miR-21, suggesting administrating n(miR-21) leads to higher miR-21 concentration in the systemic circulation within prolonged lifetime.

**Table 2 Pharmacokinetic parameters of lipo/miR-21 and n(miR-21).**

| Parameters | Lipo/miR-21 | n(miR-21) |
|---|---|---|
| *t*_{1/2}^{a} (h) | 2.19 | 9.35 |
| AUC ^{b} (µg/ml*h) | 56.9 | 403.6 |
| MRT ^{c} (h) | 1.94 | 12.70 |

| | | |
|---|---|---|
| ^{a} Elimination half-life ^{b} Area under the plasma miRNA concentration versus time curves ^{c} Mean residence time | | |

Compared to the Lipofectamine-mediated delivery, the results presented suggest that the nanocapsules lead to improved delivery efficiency into ischemic brain, decreased accumulation in the RES organs, and prolonged half-life in blood. Furthermore, because acute hepatotoxicity is a major concern for systemically delivered nanomedicines, the blood chemistry parameters that reflect the hepatic functions 24 hours post intravenous administration of the nanocapsules were also evaluated (Table 3). In contrast to the rats injected with PBS, obvious liver dysfunction was observed in the rats with Lipo/miR-21 injection; while no significant hepatotoxicity was observed for n(miR-21) even with dosage as high as 0.5 mg/kg. Because of the severe hepatotoxicity, the Lipo/miR-21 was not investigated in further *in vivo* studies.

**Table 3. Blood chemistry assay of treated rats after 24-h systemic administration of lipo/miR-21 and n(miR-21) by ELISA assay. Data are presented as mean ± SEM (n=5).**

| Group | TP (g/L) | ALB (g/L) | ALP (U/dL) | AST (U/L) | ALT (U/L) |
|---|---|---|---|---|---|
| Control | 1.55±0.02 | 0.73±0.03 | 12.19±0.59 | 60.56±4.51 | 111±7.50 |
| Lipo/miR-21 | 1.28±0.05 | 0.52±0.04 | 13.56±0.35 | 75.31±5.20 | 149.96±15.68 |
| n(miR-21) | 1.50±0.03 | 0.68±0.03 | 12.47±0.34 | 63.84±5.39 | 122.95±4.87 |

Intracarotid (*i.c.*) injection has been considered as an effect strategy to avoid accumulation of colloidal drugs in the RES and maximize bioavailability of the drug administrated. However, it also requires surgical experience with limited dosage and frequency of administration (Chen et al. (2015) Adv Drug Deliv Rev 81:128-141; Kim *et al.* (2011), *supra*)*.* In this context, intravenous (*i.v*.) injection is practically more acceptable. FIG. 12G compares the fluorescent images of both intact and excised brains after *i.c.* or *i.v* injection of n(miR-21). At low n(miR-21) dosage (0.1 mg/kg), the rat with *i.v.* injection shows less fluorescent intensity in brain tissue than that with *i.c.* injection. The fluorescent intensities become similar at a higher injection dosage (0.5 mg/kg) (FIG. 12H); further qRT-PCR results confirm similar efficacy for both i*.v*. injection and *i.c.* injection (FIG. 12I), suggesting *i.v.* injection of the nanocapsules could be an effective administration strategy for the treatment of brain ischemia.

### Therapeutic efficacy of miRNA-21 nanocapsules in model rats with transient focal cerebral ischemia.

The therapeutic efficacy of n(miR-21) was examined in rat model of transient focal cerebral ischemia induced by MCAO/R. 24 hours after creation of ischemia, n(miR-NC) and n(miR-21) were injected into the tail veins every other day for three times at a dosage of 0.5 mg/kg miRNA, respectively; while an equal volume of PBS was served as the negative control. The therapeutic benefits of n(miR-21) are mainly determined based on their neurological function and the infarct volume of the ischemic brains. The neurological findings were evaluated and scored on a five-point scale based on a double-blind test before each injection.

As shown in FIG. 14A, the average neurological deficit scores of the rats treated with either PBS or n(miR-NC) only slightly improves with time; while those treated with n(miR-21) show remarkably improved performance. Brain tissues were collected at day 7th to assess the infarct volume by 2,3,5-triphenyltetrazolium hydrochloride (TTC) staining. As shown in FIG. 14B and FIG. 14C, the TTC-stained brains of the n(miR-21)-treated rats exhibit significantly decreased infarct volume (~45%) compared to those treated with PBS or n(miR-NC). Further histopathological analysis by hematoxylin-eosin (H&E) staining of the ischemic brains shows that transient focal cerebral ischemia induced by MCAO/R resulted in obvious neuronal necrosis in the ischemic core and penumbra, which appears as eosinophilic neurons on H&E staining slices (FIG. 14D). Administration of n(miR-21) significantly protects the neurons from necrosis and improves the pathological symptoms in contrast with the PBS group and n(miR-NC) group.

To ultimately confirm the therapeutic efficacy of miR-21, in situ hybridization and immunohistochemistry were also used to analyze the expression levels of miR-21 and the downstream proteins in the ischemic boundary zone (IBZ). As shown in FIG. 14E, miR-21 expression in the IBZ was labeled by Cy3 and directly visualized in representative photomicrographs. Red fluorescence intensity from miR-21 in the brains treated by n(miR-21) is much intense than those treated with PBS or n(miR-NC), suggesting effective regulation of miR-21 levels in the IBZ by n(miR-21).

The miR-21 delivered also regulates the proteins associated with angiogenesis or neuroprotection. As confirmed by the immunohistochemistry results, expression of HIF-1α, a transcriptional factor governing the expression of vascular endothelial growth factor (VEGF) (Oladipupo et al. (2011) Proc Natl Acad Sci U S A 108:13264-13269; Semenza (2000) J Clin Invest 106:809-812) significantly increases in the brain tissues collected from n(miR-21)-treated rats in comparison with those treated with n(miR-NC) (FIG. 14F). VEGF is a crucial regulator of vascular survival and angiogenesis, and has clear potential for neural protection and regeneration (Oladipupo *et al.* (2011), *supra).* The expression of CD31, an important marker for angiogenesis (Deshpande et al. (2011) Radiology 258:804-811), as well as the level of glial fibrillary acidic protein (GFAP), which is a marker of neurologic damage48, is increased in the brain tissues of n(miR-21)-treated rats, indicating pro-angiogenic effects of the VEGF and the excellent in vivo bioactivity of n(miR-21).

Furthermore, in cerebral ischemia/reperfusion injury, delayed neuronal death is associated with apoptosis especially in the ischemic penumbra (Lee et al. (2000) J Clin Invest 106:723-731). In this study, TUNEL assay was used to detect apoptosis of neuron cells. In the slices of the brains of n(miR-21)-treated rats, significantly less green fluorescence is observed, indicating less apoptosis of the neuron cells in the IBZ (FIG. 15). It is in accordance with previous reports that upregulation of miR-21 attenuates death of neurons (Buller et al. (2010) FEBS J 277:4299-4307). Additionally, after the 7-day treatment, H&E staining of major organs reveals neither detectable pathological effects nor myocardial injury in the n(miR-21)-treated rats (FIG. 16), suggesting excellent therapeutic efficacy of n(miR-21) with satisfied safety.

In summary, a platform technology has been developed for effective systemic delivery miRNA. This technology provides new treatment of brain ischemia, a disease with leading cause of death and disability, as well as other diseases.

### References

1. Isner JM. J Clin Invest 106, 615-619 (2000).
2. Semenza GL. J Clin Invest 106, 613-614 (2000).
3. Eltzschig HK, Eckle T. Nat Med 17, 1391-1401 (2011).
4. Upadhyay RK. Biomed Res Int 2014, 869269 (2014).
5. Rosenberg GA. J Cereb Blood Flow Metab 32, 1139-1151 (2012).
6. Gállego J, Muñoz R, Martinez-Vila E. Cerebrovase Dis 27(suppl 1), 88-96 (2009).
7. Luengo-Fernandez R, Gray AM, Rothwell PM. Stroke 40, e18-23 (2009).
8. Novakovic R, Toth G, Purdy PD. J Neurointerv Surg 1, 13-26 (2009).
9. Hankey GJ. Stroke. Lancet, Preprint at http://dx.doi.org/10.1016/S0140-6736(16)30962-X (2016).
10. Lee JM, Grabb MC, Zipfel GJ, Choi DW. J Clin Invest 106, 723-731 (2000).
11. Hacke W, et al. N Engl J Med 359, 1317-1329 (2008).
12. Goyal M, et al. Stroke 47, 548 (2016).
13. Del Zoppo GJ, Saver JL, Jauch EC, Adams HP, Jr., Stroke 40, 2945-2948 (2009).
14. Emberson J, et al. Lancet 384, 1929-1935 (2014).
15. Mead GE, et al. Cochrane Database Syst Rev 11, CD009286 (2012).
16. Mortensen JK, Andersen G. Expert Opin Drug Saf 14, 911-919 (2015).
17. Prasad K, et al. Stroke 45, 3618-3624 (2014).
18. Kalladka D, et al. Lancet 388, 787-796 (2016).
19. Beavers KR, Nelson CE, Duvall CL. Adv Drug Deliv Rev 88, 123-137 (2015).
20. Inui M, Martello G, Piccolo S. Nat Rev Mol Cell Biol 11, 252-263 (2010).
21. Caputo et al. Adv Drug Deliv Rev 88, 78-91 (2015).
22. Liang TY, Lou JY. Med Sci Monit 22, 2950-2955 (2016).
23. Zhao H, et al. Stroke 44, 1706-1713 (2013).
24. Buller B, et al. FEBS J 277, 4299-4307 (2010).
25. Chen Y, Gao DY, Huang L. Adv Drug Deliv Rev 81, 128-141 (2015).
26. Kim et al. Nano Lett 11, 694-700 (2011).
27. Pecot et al. Nat Rev Cancer 11, 59-67 (2011).
28. Vickers KC, Remaley AT Curr Opin Lipidol 23, 91-97 (2012).
29. Crooke ST, et al. J Pharmacol Exp Ther 277, 923-937 (1996).
30. Cheng CJ, et al. Nature 518, 107-110 (2015).
31. Davis ME, et al. Nature 464, 1067-1070 (2010).
32. Gibbings et al. Nat Cell Biol 11, 1143-1149 (2009).
33. Yang YP, et al. Biomaterials 33, 1462-1476 (2012).
34. Chiou GY, et al. J Control Release 159, 240-250 (2012).
35. Kanasty et al. Nat Mater 12, 967-977 (2013).
36. Zhang Y, Wang Z, Gemeinhart RA. J Control Release 172, 962-974 (2013).
37. Hwang DW, et al. Biomaterials 32, 4968-4975 (2011).
38. Yan M, et al. Nat Nanotechnol 5, 48-53 (2010).
39. England CG, et al. Biomaterials 100, 101-109 (2016).
40. Chan et al. Cancer Res 65, 6029-6033 (2005).
41. Shi et al. Angew Chem Int Ed Engl 52, 3901-3905 (2013).
42. Gref R, et al. Adv Drug Deliv Rev 16, 215-233 (1995).
43. Gu Z, et al. Nano Lett 9, 4533-4538 (2009).
44. Liu C, et al. Adv Mater 27, 292-297 (2015).
45. Oladipupo S, et al. Proc Natl Acad Sci U S A 108, 13264-13269 (2011).
46. Semenza GL. J Clin Invest 106, 809-812 (2000).
47. Deshpande et al. Radiology 258, 804-811 (2011).
48. Yang Z, Wang KK. Trends Neurosci 38, 364-374 (2015).
49. Liu et al. Biomaterials 34, 817-830 (2013).
50. Pignataro et al. FEBS J 276, 46-57 (2009).

## Claims

1. A polymer nanocapsule comprising a polymer shell and a microRNA (miRNA) molecule, wherein the polymer shell comprises a) at least one positively charged monomer, b) at least one degradable cross-linker, and c) at least one neutral monomer for use in a method of treating cerebral or brain ischemia/reperfusion injury, comprising administering said polymer nanocapsule systemically, wherein the miRNA knocks down or decreases expression of an overexpressed gene and/or increases expression of a gene in which expression is otherwise reduced or inhibited, thereby treating the cerebral or brain ischemia/reperfusion injury; and
wherein the positively charged monomer is selected from N-(3-aminopropyl)methacrylamide (APM), N-(3-Aminopropyl) methacrylamide hydrochloride, acryl-spermine, dimethylamino ethyl methacrylate, (3- Acrylamidopropyl)trimethylammonium hydrochloride, (3- Acrylamidopropyl)trimethylammonium hydrochloride, N-(3-((4-((3-aminopropyl)amino)butyl)amino)propyl)methacrylamide, N-(3-((4-aminobutyl)amino)propyl)acrylamide, N-(3-((4-aminobutyl)amino)propyl)methacrylamide, N-(2-((2-aminoethyl)(methyl)amino)ethyl)methacrylamide, N-(piperazin-1-ylmethyl)acrylamide, N-(piperazin-1-ylmethyl)methacrylamide, N-(2-(bis(2-aminoethyl)amino)ethyl)acrylamide, and N-(2-(bis(2-aminoethyl)amino)ethyl)methacrylamide; and preferably wherein the positively charged monomer is N-(3-aminopropyl)methacrylamide (APM);
wherein the degradable cross-linker comprises a crosslinker selected from glycerol dimethacrylate (GDMA), 1,3-glycerol dimethacrylate, glycerol 1,3-diglycerolate diacrylate, N,N'-bis(acryloyl)cysteamine, bis[2-(methacryloyloxy)ethyl]phosphate, and bisacryloylated polypeptide; and preferably wherein the degradable cross- linker comprises glycerol dimethacrylate (GDMA); and
wherein the neutral monomer is selected from acrylamide (AAM), poly(ethylene glycol) methyl ether acrylate (mPEG), or a combination thereof.

2. The polymer nanocapsule for use of claim 1, wherein the degradable cross- linker is glycerol dimethacrylate (GDMA); the positively charged monomer is N-(3-aminopropyl)methacrylamide (APM); and the neutral monomer is acrylamide (AAM), poly(ethylene glycol) methyl ether acrylate (mPEG), or a combination thereof.

3. The polymer nanocapsule for use of any one of the preceding claims, wherein the molar ratio of the positively charged monomer and the neutral monomer is at least about 1:1, about 1:2, about 1:3, about 1:4, about 1:5, about 1:6, about 1:7, about 1:8, about 1:9, about 1:10, about 1:11, about 1:12, about 1:13, about 1:14, about 1:15, about 1:20, and preferably at least about 1:10 or about 1:11.

4. The polymer nanocapsule for use of claim 2, wherein the molar ratio of APM: mPEG is at least about 5:1, about 5:2, about 5:3, about 5:4, about 5:5, and preferably at least about 5:1 or about 5:2; and wherein the molar ratio of GDMA:APM is at least about 2:1, 1:1, 1:2 or more, preferably at least about 1:1.

5. The polymer nanocapsule for use of any one of the preceding claims, wherein the miRNA is selected from a miRNA that modulates genes which induce decreased apoptosis, increased angiogenesis, increased neurogenesis, or increased neuroplasticity.

6. The polymer nanocapsule for use of any one of the preceding claims, wherein the miRNA is selected from a miRNA that increases the expression of at least one of VEGF, GFAP, CD31, CD34, BCL-2, NeuN, bromodeoxyuridine, nestin, PSA-NCAM, doublecortin, Pax6, GAP-43, AKT, or HIF1-a.

7. The polymer nanocapsule for use of any one of the preceding claims, wherein the polymer nanocapsule is degradable in late endosomes.

8. The polymer nanocapsule for use of any one of the preceding claims, further conjugated to an agent, wherein the agent is a labeling agent, a targeting agent or a combination thereof.

9. The polymer nanocapsule for use of claim 8, wherein the targeting agent is selected from a TAT (transduction domain of human immunodeficiency virus type-1 (HIV-1) peptide, a diphtheria toxin, a tetanus toxin, Tet1, capsid protein G23, a rabies virus glycoprotein (RVG) peptide, an opioid peptide, glutathione, thiamine, leptin, an angiopep, a low-density lipoprotein, insulin, melanotransferrin, and transferrin.

## Patentansprüche

1. Polymer-Nonokapsel, umfassend eine Polymerhülle und ein microRNA (miRNA)-Molekül, wobei die Polymerhülle a) mindestens ein positiv geladenes Monomer, b) mindestens einen abbaubaren Vernetzer, und c) mindestens ein neutrales Monomer umfasst zur Verwendung in einem Verfahren zur Behandlung von zelebraler oder Gehirn-Ischämie/Reperfusionsschaden, umfassend systemisches Verabreichen der Polymer-Nanokapsel, wobei die miRNA die Expression eines überexprimierten Gens unterdrückt oder verringert und/oder die Expression eines Gens steigert, dessen Expression anderweitig verringert oder gehemmt ist, wodurch die zerebrale oder Gehirn-Ischämie / der Reperfusionschaden behandelt wird; und
wobei das positiv geladene Monomer ausgewählt ist aus N-(3-Aminopropyl)methacrylamid (APM), N-(3-Aminopropyl)methacrylamid-Hydrochlorid, Acryl-Spermin, Dimethylaminoethylmethacrylat, (3-Acrylamidopropyl)trimethylammonium-Hydrochlorid, (3-Acrylamidopropyl)trimethylammonium-Hydrochlorid, N-(3-((4-((3-aminopropyl)amino)butyl)amino)propyl)methacrylamid, N-(3-((4-Aminobutyl)amino)propyl)acrylamid, N-(3-((4-Aminobutyl)amino)propyl)methacrylamid, N-(2-((2-Aminoethyl)(methyl)amino)ethyl)methacrylamid, N-(Piperazin-1-ylmethyl)acrylamid, N-(Piperazin-1-ylmethyl)methacrylamid, N-(2-(Bis(2-aminoethyl)amino)ethyl)acrylamid und N-(2-(Bis(2-aminoethyl)amino)ethyl)methacrylamid; und wobei vorzugsweise das positiv geladene Monomer N-(3-aminopropyl)methacrylamid (APM) ist;
wobei der abbaubare Vernetzer einen Vernetzer ausgewählt aus Glycerindimethacrylat (GDMA), 1,3-Glycerindimethacrylat, Glycerin-1,3-diglycerolatdiacrylat, N,N' Bis(acryloyl)cysteamin, Bis[2-(methacryloyloxy)ethyl]phosphat und bisacryloyliertes Polypeptid umfasst, und wobei vorzugsweise der abbaubare Vernetzer Glycerindimethacrylat (GDMA) umfasst; und
wobei das neutrale Monomer ausgewählt ist aus Acrylamid (AAM), Poly(ethylenglykol)methyletheracrylat (mPEG) oder einer Kombination davon.

2. Polymer-Nanokapsel zur Verwendung nach Anspruch 1, wobei der abbaubare Vernetzer Glycerindimethacrylat (GDMA) ist; das positiv geladene Monomer N-(3-Aminopropyl)methacrylamid (APM) ist; und das neutrale Monomer Acrylamid (AAM), Poly(ethylenglykol)methyletheracrylat (mPEG) oder eine Kombination davon ist.

3. Polymer-Nanokapsel zur Verwendung nach einem der vorhergehenden Ansprüche, wobei das molare Verhältnis des positiv geladenen Monomers und des neutralen Monomers mindestens etwa 1:1, etwa 1:2, etwa 1:3, etwa 1:4, etwa 1:5, etwa 1:6, etwa 1:7, etwa 1:8, etwa 1:9, etwa 1:10, etwa 1:11, etwa 1:12, etwa 1:13, etwa 1:14, etwa 1:15, etwa 1:20 und vorzugsweise mindestens etwa 1:10 oder etwa 1:11 beträgt.

4. Polymer-Nanokapsel zur Verwendung nach Anspruch 2, wobei das molare Verhältnis von APM: mPEG mindestens etwa 5:1, etwa 5:2, etwa 5:3, etwa 5:4, etwa 5:5 und vorzugsweise mindestens etwa 5:1 oder etwa 5:2 beträgt; und wobei das molare Verhältnis von GDMA:APM mindestens etwa 2:1, 1:1, 1:2 oder mehr, vorzugsweise mindestens etwa 1:1 beträgt.

5. Polymer-Nanokapsel zur Verwendung nach einem der vorhergehenden Ansprüche, wobei die miRNA aus einer miRNA ausgewählt ist, die Gene moduliert, die eine verringerte Apoptose, eine erhöhte Angiogenese, eine erhöhte Neurogenese oder eine erhöhte Neuroplastizität induzieren.

6. Polymer-Nanokapsel zur Verwendung nach einem der vorhergehenden Ansprüche, wobei die miRNA aus einer miRNA ausgewählt ist, die die Expression von mindestens einem aus VEGF, GFAP, CD31, CD34, BCL-2, NeuN, Bromdesoxyuridin, Nestin, PSA-NCAM, Doublecortin, Pax6, GAP-43, AKT oder HIF1-a steigert.

7. Polymer-Nanokapsel zur Verwendung nach einem der vorhergehenden Ansprüche, wobei die Polymer-Nanokapsel in späten Endosomen abbaubar ist.

8. Polymer-Nanokapsel zur Verwendung nach einem der vorhergehenden Ansprüche, die weiterhin mit einem Mittel konjugiert ist, wobei das Mittel ein Markierungsmittel, ein Targeting-Mittel oder eine Kombination davon ist.

9. Polymer-Nanokapsel zur Verwendung nach Anspruch 8, wobei das Targeting-Mittel ausgewählt ist aus einem TAT (Transduktionsdomäne des humanen Immundefizienzvirus Typ-1 (HIV-1))-peptid, einem Diphtherietoxin, einem Tetanustoxin, Tet1, dem Capsidprotein G23, einem Tollwutvirus-Glykoprotein (RVG)-Peptid, einem Opioid-Peptid, Glutathion, Thiamin, Leptin, einem Angiopep, einem Low-Density-Lipoprotein, Insulin, Melanotransferrin und Transferrin.

## Revendications

1. Nanocapsule polymérique comprenant une coque en polymère et une molécule microARN (miARN), dans laquelle la coque en polymère comprend a) au moins un monomère chargé positivement, b) au moins un agent de réticulation dégradable et c) au moins un monomère neutre, pour son utilisation dans une méthode pour traiter des lésions cérébrales ou d'ischémie-reperfusion du cerveau, comprenant l'administration systémique de ladite nanocapsule polymérique, dans laquelle le miARN inhibe ou diminue l'expression d'un gène surexprimé et/ou augmente l'expression d'un gène dont l'expression est autrement réduite ou inhibée, traitant ainsi les lésions cérébrales ou d'ischémiereperfusion du cerveau; et
dans laquelle le monomère chargé positivement est choisi parmi le N-(3-aminopropyl)méthacrylamide (APM), le chlorhydrate de N-(3-aminopropyl)méthacrylamide, un acyle-spermine, le méthacrylate de diéthylaminoéthyle, le chlorhydrate de (3-acrylamidopropyl)triméthylammonium, le chlorhydrate de (3-acrylamidopropyl)triméthylammonium, le N-(3-((4-((3-aminopropyl)amino)butyl)amino)propyl)méthacrylamide, le N-(3-((4-aminobutyl)amino)propyl)acrylamide, le N-(3-((4-aminobutyl)amino)propyl)methacrylamide, le N-(2-((2-aminoéthyl)(méthyl)amino)éthyl)méthacrylamide, le N-(pipérazin-1-ylméthyl)acrylamide, le N-(pipérazin-1-ylméthyl)méthacrylamide, le N-(2-(bis(2-aminoéthyl)amino)éthyl)acrylamide, et le N-(2-(bis(2-aminoéthyl)amino)éthyl)méthacrylamide, et de préférence dans laquelle le monomère chargé positivement est le N-(3-aminopropyl)méthacrylamide (APM);
dans laquelle l'agent de réticulation dégradable comprend un agent de réticulation choisi parmi le diméthacrylate de glycérol (GDMA), le diméthacrylate de 1,3-glycérol, le 1,3-diglycérolate diacrylate de glycérol, la N,N'-bis(acryloyl)cystéamine, le bis[2-(méthacryloyloxy)éthyl]phosphate, et un polypeptide bisacryloylé, et de préférence dans laquelle l'agent de réticulation dégradable comprend du diméthacrylate de glycérol (GDMA); et
dans laquelle le monomère neutre est choisi parmi l'acrylamide (AAM), l'acrylate d'éther méthylique de polyéthylène glycol (mPEG) ou une combinaison de ceux-ci.

2. Nanocapsule polymérique pour son utilisation selon la revendication 1, dans laquelle l'agent de réticulation dégradable est du diméthacrylate de glycérol (GDMA); le monomère chargé positivement est du N-(3-aminopropyl)méthacrylamide (APM); et le monomère neutre est de l'acrylamide (AAM), de l'acrylate d'éther méthylique de polyéthylène glycol (mPEG) ou une combinaison de ceux-ci.

3. Nanocapsule polymérique pour son utilisation selon l'une quelconque des revendications précédentes, dans laquelle le rapport molaire du monomère chargé positivement au monomère neutre est d'au moins environ 1:1, environ 1:2, environ 1:3, environ 1:4, environ 1:5, environ 1:6, environ 1:7, environ 1:8, environ 1:9, environ 1:10, environ 1:11, environ 1:12, environ 1:13, environ 1:14, environ 1:15, environ 1:20, et de préférence d'au moins environ 1:10 ou environ 1:11.

4. Nanocapsule polymérique pour son utilisation selon la revendication 2, dans laquelle le rapport molaire de APM:mPEG est d'au moins environ 5:1, environ 5:2, environ 5:3, environ 5:4, environ 5:5, et de préférence d'au moins environ 5:1 ou environ 5:2; et dans laquelle le rapport molaire de GDMA:APM est d'au moins environ 2:1, 1:1, 1:2 ou plus, de préférence d'au moins environ 1:1.

5. Nanocapsule polymérique pour son utilisation selon l'une quelconque des revendications précédentes, dans laquelle le miARN est choisi parmi un miARN qui module des gènes qui induisent une diminution de l'apoptose, une augmentation de l'angiogenèse, une augmentation de la neurogénèse ou une augmentation de la neuroplasticité.

6. Nanocapsule polymérique pour son utilisation selon l'une quelconque des revendications précédentes, dans laquelle le miARN est choisi parmi un miARN qui augmente l'expression d'au moins l'un parmi le VEGF, le GFAP, le CD31, le CD34, le BCL-2, le NeuN, la bromodésoxyuridine, la nestine, le PSA-NCAM, la doublecortine, le Pax6, le GAP-43, l'AKT ou le HIF1-a.

7. Nanocapsule polymérique pour son utilisation selon l'une quelconque des revendications précédentes, dans laquelle la nanocapsule polymérique est dégradable dans les endosomes tardifs.

8. Nanocapsule polymérique pour son utilisation selon l'une quelconque des revendications précédentes, en outre conjuguée à un agent, dans laquelle l'agent est un agent de marquage, un agent de ciblage ou une combinaison de ceux-ci.

9. Nanocapsule polymérique pour son utilisation selon la revendication 8, dans lequel l'agent de ciblage est choisi parmi un peptide TAT (domaine de transduction du virus de l'immunodéficience humaine de type 1 (VIH-1)), une toxine diphtérique, une toxine tétanique, Tet1, une protéine de la capside G23, un peptide d'une glycoprotéine du virus de la rage (RVG), un peptide opioïde, le glutathion, le thiamine, la leptine, un angiopep, une lipoprotéine de basse densité, l'insuline, la mélanotransferrine et la transferrine.
